(19) **Europäisches Patentamt European Patent Office Office européen des brevets**

(11) **EP 4 736 865 A1**

(12) **EUROPEAN PATENT APPLICATION**
published in accordance with Art. 153(4) EPC

(43) Date of publication:
06.05.2026  Bulletin 2026/19

(21) Application number: 24839675.6

(22) Date of filing: 05.07.2024

(51) International Patent Classification (IPC):
$A61K\ 38/00^{(2006.01)}$    $A61K\ 38/19^{(2006.01)}$
$A61K\ 38/43^{(2006.01)}$    $A61K\ 39/35^{(2006.01)}$
$A61K\ 39/395^{(2006.01)}$    $A61K\ 47/16^{(2006.01)}$
$A61K\ 47/18^{(2017.01)}$    $A61K\ 47/26^{(2006.01)}$
$A61P\ 43/00^{(2006.01)}$

(52) Cooperative Patent Classification (CPC):
A61K 38/00; A61K 38/19; A61K 38/43;
A61K 39/35; A61K 39/395; A61K 47/16;
A61K 47/18; A61K 47/26; A61P 43/00

(86) International application number:
PCT/JP2024/024412

(87) International publication number:
WO 2025/013783 (16.01.2025 Gazette 2025/03)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 07.07.2023  JP 2023112438

(71) Applicant: Nagase Viita Co., Ltd.
Okayama-shi, Okayama 702-8006 (JP)

(72) Inventors:
• KONO, Keizo
  Okayama-shi, Okayama 702-8006 (JP)

• KUSANO, Hajime
  Okayama-shi, Okayama 702-8006 (JP)
• MITSUKAWA, Yuki
  Okayama-shi, Okayama 702-8006 (JP)
• HASHIMOTO, Kotaro
  Okayama-shi, Okayama 702-8006 (JP)
• HOJO, Yukiko
  Okayama-shi, Okayama 702-8006 (JP)

(74) Representative: Manitz Finsterwald
Patent- und Rechtsanwaltspartnerschaft mbB
Martin-Greif-Straße 1
80336 München (DE)

(54) **COMPOSITION FOR INHIBITING DENATURATION OF PROTEIN**

(57) The present invention provides a composition for inhibiting protein denaturation, capable of inhibiting denaturation of a wide range of proteins. The present invention relates to a composition for inhibiting protein denaturation, containing at least one saccharide selected from the group consisting of trehalose, sucrose, maltitol, and isomaltitol and at least one amine compound selected from the group consisting of glutamic acid, aspartic acid, arginine, proline, serine, choline, and salts thereof.

**EP 4 736 865 A1**

**Description**

TECHNICAL FIELD

The present invention relates to a composition for inhibiting protein denaturation.

BACKGROUND ART

Proteins are widely used in fields such as therapeutic agents, diagnostic agents, vaccines, and food. However, proteins alone are prone to denaturation, and when subjected to heating, long-term storage, multiple freeze-thaw cycles, or other processes, they become denatured and lose their function. Efforts to improve protein-containing compositions are being made to inhibit protein denaturation.

Carbohydrates and amino acids have been known to inhibit protein denaturation. For example, Patent Literature 1 discloses a method for stabilizing a Fc-mutant protein using a carbohydrate, a cationic amino acid, an anion, and a polysorbate. Patent Literature 2 discloses a method for stabilizing an anti-TNFα antibody using a polyol, a surfactant, and arginine. These methods can stabilize only limited types of proteins.

CITATION LIST

- Patent Literature

Patent Literature 1: JP 2009-525986 T
Patent Literature 2: JP 2022-097600 A

SUMMARY OF INVENTION

- Technical Problem

An object of the present invention is to provide a composition for inhibiting protein denaturation, capable of inhibiting denaturation of a wide range of proteins.

- Solution to Problem

The present inventors have found that a combination of a specific saccharide and a specific amine compound enables the inhibition of protein denaturation. Based on this finding, the present invention has been completed.

Specifically, the present invention relates to a composition for inhibiting protein denaturation, containing at least one saccharide selected from the group consisting of trehalose, sucrose, maltitol, and isomaltitol and at least one amine compound selected from the group consisting of glutamic acid, aspartic acid, arginine, proline, serine, choline, and salts thereof.

Preferably, the composition for inhibiting protein denaturation inhibits protein denaturation caused by heating or freeze-thawing.

Preferably, the composition for inhibiting protein denaturation inhibits protein aggregation.

Preferably, the protein is at least one selected from the group consisting of antibodies, cytokines, allergens, and enzymes.

Preferably, the protein is at least one selected from the group consisting of anti-IFNα antibodies, anti-IFNγ antibodies, anti-TNFα antibodies, anti-IL-17A antibodies, and anti-IL-18 antibodies.

Preferably, the protein is at least one selected from the group consisting of IFNα, IFNγ, IL-18, and TNFα.

Preferably, the protein is a cedar pollen allergen.

Preferably, the protein is at least one selected from the group consisting of lactate dehydrogenases, glycosyltransferases, saccharide-degrading enzymes, malate dehydrogenases, and protein-degrading enzymes.

Preferably, the protein is a virus-derived protein or a bacteria-derived protein.

Preferably, the protein is a blood coagulation factor or albumin.

Preferably, a molar ratio of the saccharide to the amine compound in the composition for inhibiting protein denaturation is from 40:1 to 1:40.

Preferably, the composition for inhibiting protein denaturation is in liquid form, and a total concentration of the saccharide and the amine compound in the composition is from 0.20 mol/L to 3.50 mol/L.

Preferably, the composition for inhibiting protein denaturation inhibits protein denaturation synergistically compared to a protein denaturation inhibitory effect of the saccharide and a protein denaturation inhibitory effect of the amine compound.

The present invention also relates to a method for inhibiting protein denaturation, including allowing a protein to coexist with the composition for inhibiting protein denaturation.

The present invention also relates to a protein formulation, containing a protein and the composition for inhibiting protein denaturation.

The present invention also relates to a method for inhibiting protein denaturation, including allowing a protein to coexist with:

at least one saccharide selected from the group consisting of trehalose, sucrose, maltitol, and isomaltitol, and
at least one amine compound selected from the group consisting of glutamic acid, aspartic acid, arginine, proline, serine, choline, and salts thereof.

The present invention also relates to a protein formulation, containing:

at least one saccharide selected from the group consisting of trehalose, sucrose, maltitol, and isomaltitol;
at least one amine compound selected from the group consisting of glutamic acid, aspartic acid, arginine, proline, serine, choline, and salts thereof; and
a protein.

- Advantageous Effects of Invention

The composition for inhibiting protein denaturation according to the present invention can inhibit denaturation of a wide range of proteins.

DESCRIPTION OF EMBODIMENTS

<<Composition for inhibiting protein denaturation>>

The present invention relates to a composition for inhibiting protein denaturation, containing at least one saccharide selected from the group consisting of trehalose, sucrose, maltitol, and isomaltitol and at least one amine compound selected from the group consisting of glutamic acid, aspartic acid, arginine, proline, serine, choline, and salts thereof. Due to the combination of the saccharide and the amine compound, the composition can inhibit denaturation of a wide range of proteins.

<Saccharide>

The saccharide in the present invention is selected from the group consisting of trehalose, sucrose, maltitol, and isomaltitol. These saccharides may be used alone, or two or more of these may be used in combination. Trehalose, sucrose, and maltitol are preferred among these, with trehalose being more preferred. Moreover, each of these saccharides may be a derivative obtained by introducing a substituent into the hydrogen atoms of some of the hydroxy groups. Examples of such substituents include carboxylic acid esters such as acetate and benzoate; sulfuric acid esters; fatty acid esters such as laurate, myristate, palmitate, stearate, oleate, linoleate, and linolenate; and ethers such as methyl ether, benzyl ether, trityl ether, methyl silyl ether, and dodecyl ether.

The saccharide concentration in the composition for inhibiting protein denaturation is preferably 0.10 to 3.00 mol/L, more preferably 0.40 to 1.60 mol/L, still more preferably 0.60 to 1.20 mol/L.

When the composition for inhibiting protein denaturation is mixed with a protein to obtain a protein formulation, the saccharide concentration in the protein formulation is preferably 0.10 to 3.00 mol/L, more preferably 0.40 to 1.60 mol/L, still more preferably 0.60 to 1.20 mol/L.

In the present invention, the saccharide acting as a hydrogen bond donor and the amine compound acting as a hydrogen bond receptor may form a natural deep eutectic solvent (NADES) to inhibit protein denaturation for protein stabilization, although the present invention is not limited to this mechanism.

<Amine compound>

The amine compound in the present invention is selected from the group consisting of glutamic acid, aspartic acid, arginine, proline, serine, choline, and salts thereof. Examples of salts that may constitute the amine compound include sodium salts, potassium salts, hydrochlorides, and chlorides.

These amine compounds may be used alone, or two or more of these may be used in combination. Glutamic acid, aspartic acid, arginine, proline, serine, and salts thereof are preferred among these, with glutamic acid, aspartic acid, arginine, and salts thereof being more preferred. In the present invention, the amine compound may act as a hydrogen bond receptor to form a NADES, although the present invention is not limited to this mechanism.

Although the combination of the saccharide and the amine compound is not limited, it is preferably a combination of trehalose and an amine compound selected from the group consisting of arginine, aspartic acid, serine, and glutamic acid, or a salt thereof; a combination of sucrose and an amine compound selected from the group consisting of choline, arginine, and glutamic acid, or a salt thereof; a combination of maltitol and an amine compound selected from the group consisting of arginine, proline, and glutamic acid, or a salt thereof; or a combination of isomaltitol and arginine or a salt thereof. The combination is more preferably a combination of maltitol and potassium glutamate or sodium glutamate, a combination of trehalose and potassium glutamate or sodium glutamate, a combination of trehalose and potassium aspartate or sodium aspartate, or a combination of maltitol and arginine hydrochloride. The combination is still more preferably a combination of maltitol and sodium glutamate, a combination of trehalose and sodium glutamate, or a combination of trehalose and sodium aspartate. These combinations can achieve a larger effect in inhibiting protein denaturation.

The amine compound concentration in the composition for inhibiting protein denaturation is preferably 0.06 to 3.30 mol/L, more preferably 0.13 to 2.60 mol/L, particularly preferably 0.21 to 1.90 mol/L.

Moreover, when the composition for inhibiting protein denaturation is mixed with a protein to obtain a protein formulation, the amine compound concentration in the protein formulation is preferably 0.06 mol/L to 3.30 mol/L% by weight, more preferably 0.13 to 2.60 mol/L, particularly preferably 0.21 to 1.90 mol/L.

In the composition for inhibiting protein denaturation, the molar ratio of the saccharide to the amine compound is preferably from 40:1 to 1:40, more preferably from 20:1 to 1:20, still more preferably from 10:1 to 1:10. Here, if the amount of the saccharide is either larger or smaller than that corresponding to a molar ratio of the saccharide to the amine compound ranging from 40:1 to 1:40, no protein denaturation inhibitory effect tends to be found due to the molar saccharide concentration being too high or too low.

When the composition for inhibiting protein denaturation is in liquid form, the total concentration of the saccharide and the amine compound is preferably from 0.20 to 3.50 mol/L, more preferably from 0.80 to 3.00 mol/L, still more preferably from 1.00 to 2.50 mol/L. When the composition for inhibiting protein denaturation is in solid form, the total concentration of the saccharide and the amine compound is preferably from 0.05 to 3.50 mol/kg, more preferably from 0.10 to 2.00 mol/kg, still more preferably from 0.20 to 1.50 mol/kg.

<Form of composition for inhibiting denaturation>

The composition for inhibiting protein denaturation may be in any form and may be either liquid or solid. The liquid form may be an aqueous solution, a suspension, a slurry, or another type of liquid form. The solid form may be powder, granules, a tablet, or another type of solid form. Among these, the composition is preferably in liquid form, in view of ease of production and storage stability.

The composition for inhibiting protein denaturation, which is in liquid form, preferably has a pH of 4 to 9, more preferably 5 to 8. The above pH conditions are less likely to cause precipitation and exert less influence on protein activity. The pH of the composition for inhibiting protein denaturation can be controlled using an acid, such as hydrochloric acid or sulfuric acid, or a base, such as sodium hydroxide or potassium hydroxide.

The composition for inhibiting protein denaturation can be prepared by mixing the components in any order. After mixing the components, the mixture may be subjected to filtration or microorganism elimination by bringing it into contact with a porous material or by passing it through a filter.

<Optional component>

In addition to the saccharide and the amine compound, the composition for inhibiting protein denaturation may contain optional components such as a pH adjuster, a surfactant, a thickener, an emulsifier, an inorganic salt, a preservative, a solvent, an excipient, a metal, or a filter aid. The amount of these optional components is not limited and may be appropriately selected by a person skilled in the art. For example, it may be 0.001 to 90% by weight, preferably 0.01 to 50% by weight, more preferably 0.1 to 30% by weight, based on the composition for inhibiting protein denaturation.

Examples of pH adjusters include ascorbic acid, acetic acid, dehydroacetic acid, lactic acid, citric acid, gluconic acid, succinic acid, tartaric acid, fumaric acid, malic acid, and adipic acid, and sodium (Na) salts, calcium (Ca) salts, and potassium (K) salts of these organic acids, as well as carbonic acid, phosphoric acid, and pyrophosphoric acid, and Na salts and K salts of these inorganic acids.

Examples of surfactants include nonionic surfactants, anionic surfactants, and cationic surfactants. Specific examples of the nonionic surfactants include polysorbate 20, polysorbate 80, polyoxyethylene sorbitan fatty acid esters, sorbitan monolaurate, sorbitan monooleate, polyoxyethylene polyoxypropylene glycol, and polyoxyethylene sorbitan monooleate. Specific examples of the anionic surfactants include dioctanoyl sodium sulfate and sodium lauryl sulfate. Nonionic surfactants are preferred among these, with polysorbate 20 or polysorbate 80 being more preferred. The surfactant concentration in the composition for inhibiting protein denaturation is preferably 0.005 to 5 (w/v)%, more preferably 0.01 to 3 (w/v)%, still more preferably 0.05 to 1 (w/v)%.

Examples of thickeners/excipients include gums, alginic acid, alginic acid derivatives, pectin, carrageenan, curdlan, pullulan, gelatin, cellulose derivatives, agar, tamarind, psyllium, glucomannan, polyethylene glycol, mineral oils, and silicone oils.

Examples of emulsifiers include glycerol fatty acid esters, polyglycerol fatty acid esters, sucrose fatty acid esters, propylene glycol fatty acid esters, sorbitan fatty acid esters, lecithin, enzyme-degraded lecithin, and saponin.

Examples of inorganic salts include sodium chloride, ammonium sulfate, sodium sulfate, calcium chloride, and polyphosphates.

Examples of preservatives include propionic acid, propionates, sulfites, benzoates, sorbic acid, sorbates, polylysine, glycine, and acetates. Examples of the foregoing salts include sodium (Na), calcium (Ca), and potassium (K) salts.

Examples of solvents include water, ethanol, methanol, and isopropanol. The solvents may contain a buffer component such as phosphoric acid, citric acid, glycine, or Tris.

Examples of excipients include hyaluronic acid, methylcellulose, carboxymethyl cellulose, hydroxypropyl methylcellulose, poly(vinyl alcohol), polyethylene glycol, triglycerides, glycerides, propylene glycol, and polyvinylpyrrolidone.

<Evaluation of inhibition of protein denaturation>

The inhibition of protein denaturation can be evaluated by the following method. Specifically, a protein formulation containing the composition for inhibiting protein denaturation according to the present invention is denatured, and then the protein activity (A) is measured. Separately, a control sample is denatured in the same manner, and then the protein activity (B) is measured. Then, it can be considered that the inhibition of protein denaturation is achieved if A exceeds B. The control sample may be a formulation that contains the same protein as that in the test sample but does not contain the composition for inhibiting denaturation.

The treatment for denaturing the protein formulation in the evaluation of inhibition of protein denaturation may be heating, freeze-thawing, or another treatment. The heating conditions preferably include a temperature of 30°C to 100°C, more preferably 35°C to 75°C, still more preferably 37°C, 40°C, 70°C, or 73°C. The duration of heating is preferably one hour to three days, more preferably one to 24 hours, still more preferably one to six hours, further preferably two hours or five hours. The freeze-thawing conditions include, for example, ten repeated cycles of freezing at -80°C for 1.5 to 2 hours and thawing at room temperature (about 20°C).

The protein activity may be measured by, for example, a method utilizing an antigen-antibody reaction, such as EIA or ELISA, a protein aggregation assay, or a method for measurement of protein enzymatic activity. When the protein activity is measured using competitive EIA or competitive ELISA, the competitive inhibition rate (%) is measured as the protein activity.

When the protein activity is measured by a method utilizing an antigen-antibody reaction, such as EIA or ELISA, the ratio (A/B) of the protein activity (A) to the protein activity (B) is higher than 1, preferably 1.05 or higher, more preferably 1.1 or higher, still more preferably 1.2 or higher, further preferably 1.5 or higher, particularly preferably 2 or higher, 5 or higher, 10 or higher, or 100 or higher.

When the protein activity is measured by a protein aggregation assay, the ratio (A/B) of the protein activity (A) to the protein activity (B) is preferably lower than 1, more preferably 0.5 or lower, still more preferably 0.3 or lower, further preferably 0.1 or lower.

When the protein activity is evaluated by enzymatic activity, the ratio (A/B) of the protein activity (A) to the protein activity (B) is higher than 1, preferably 1.05 or higher, more preferably 1.1 or higher, still more preferably 1.2 or higher, further preferably 1.5 or higher, particularly preferably 2 or higher, 5 or higher, 10 or higher, or 100 or higher.

The composition for inhibiting protein denaturation preferably inhibits protein denaturation synergistically compared to the protein denaturation inhibitory effect of the saccharide and the protein denaturation inhibitory effect of the amine compound.

Whether the protein denaturation is inhibited synergistically can be evaluated by the following method. Specifically, a protein formulation containing the composition for inhibiting protein denaturation according to the present invention is denatured, and then the protein activity (A) is measured. Separately, a formulation that contains the same protein and the same saccharide as those in the test sample but does not contain any amine compound is prepared as a control sample 1 and denatured in the same manner, and then the protein activity ($B_1$) is measured. A formulation that contains the same protein and the same amine compound as those in the test sample but does not contain any saccharide is prepared as a control sample 2 and denatured in the same manner, and then the protein activity ($B_2$) is measured. Then, it can be considered that the inhibition of protein denaturation is synergistic if $A > B_1$ and $A > B_2$. Preferably, $A \geq B_1 \times 1.5$ and $A \geq B_2 \times 1.5$, more preferably $A \geq B_1 \times 2$ and $A \geq B_2 \times 2$, still more preferably $A \geq B_1 + B_2$, further preferably $A \geq (B_1 + B_2) \times 2$, particularly preferably $A \geq (B_1 + B_2) \times 10$.

The composition for inhibiting protein denaturation can inhibit protein denaturation, even when subjected to the above-described denaturation treatment, and therefore enables long-term stable storage of proteins under mild conditions that hardly cause denaturation. The protein formulation containing the composition for inhibiting protein denaturation according to the present invention is preferably stored at a temperature of 4°C to 40°C. The protein formulation is preferably stored at a relative temperature of 0% to 75%. Moreover, the storage period of the protein formulation is preferably one year or longer, more preferably two years or longer, at 4°C.

<<Protein formulation>>

A protein formulation according to the present invention contains a protein and the composition for inhibiting protein denaturation.

<Protein>

In the present invention, the protein whose denaturation is to be inhibited is not limited. It is possible to inhibit the denaturation of a protein that is generally used as, for example, an antibody, a cytokine, an allergen, an enzyme, a blood coagulation and fibrinolysis factor, or a hormone.

The antibody may be any antibody that can specifically bind to an antigen, and may be any of IgG, IgA, IgM, IgD, and IgE. Moreover, the antibody may be derived from any animal, examples of which include humans and non-human animals such as mice, rats, rabbits, sheep, goats, and chickens. The antibody may also be a chimeric antibody or humanized antibody combining a sequence derived from a non-human animal with a sequence derived from a human, or may be a fully human antibody. Examples of the antibody also include antibodies containing both complete constant regions and complete variable regions, as well as Minibody, Fab, Diabody, Fab', Triabody, scFv-Fc, scFv, F(ab')$_2$, and Fv. The antibody may also be an antibody conjugated with a compound other than antibodies, such as a nucleic acid, a labeling compound, or a small molecule drug. The antigen that the antibody specifically binds to is not limited and may be a nucleic acid, a protein, or a saccharide, for example. Specific examples of the antigen include IFN$\alpha$, IFN$\gamma$, TNF$\alpha$, IL-18, IL-17, and leukocyte surface antigen proteins.

Examples of the cytokine include IFNs such as IFN$\alpha$, IFN$\beta$, IFN$\gamma$, IFN$\delta$, IFN$\omega$, IFN$\lambda$, IFN$\epsilon$, and IFNK; TNFs such as TNFN$\alpha$ and TNF$\beta$; interleukins (ILs) such as IL1, IL2, IL3, IL4, IL5, IL6, IL7, IL8, IL9, IL10, IL11, IL12, IL13, IL14, IL15, IL16, IL17, and IL18; CSF, EPO, EGF, FGF, and PDGF.

The allergen may be any material that can cause allergies in humans or non-human animals. Examples include food allergens such as shrimp, crab, walnut, wheat, buckwheat, egg, milk, peanut, almond, abalone, squid, salmon roe, orange, cashew nut, kiwi fruit, beef, sesame, salmon, mackerel, soybean, chicken, banana, pork, matsutake mushroom, peach, Japanese yam, apple, and gelatin; and pollen allergens such as cedar pollen, ragweed pollen, Japanese pampas grass pollen, and Japanese red pine pollen.

The enzyme may be any protein that has catalytic activity for a chemical reaction. Examples include saccharide-degrading enzymes such as $\alpha$-amylase, $\beta$-amylase, glucoamylase, isoamylase, maltogenic amylase, pullulanase, maltotriohydrolase, dextranase, glucose isomerase, cellulase, xylanase, hemicellulase, mannanase, pectinase, pectin methylesterase, invertase, lactase, inulinase, $\alpha$-galactosidase, chitinase, chitosanase, alginate lyase, glucose oxidase, and glucose dehydrogenase; glycosyltransferases such as cyclodextrin glucanotransferase, transglucosidase, 6-$\alpha$-glucanotransferase, glucosyltransferase, galactosyltransferase, sialyltransferase, N-acetylglucosaminyltransferase, and mannosyltransferase; protein-degrading enzymes such as protease, peptidase, and collagenase; amino acid-degrading enzymes such as glutaminase; lipid-related enzymes such as lipase, phospholipase, and esterase; and other enzymes such as catalase, urease, tannase, deaminase, alcohol dehydrogenase, lactate dehydrogenase (LDH), malate dehydrogenase, lysosome, alkaline phosphatase, DNA-degrading enzymes, and uric acid oxidase.

The protein concentration in the protein formulation is preferably 0.0001 to 100 mg/mL, more preferably 0.001 to 50 mg/mL, still more preferably 0.01 to 30 mg/mL. The protein concentration may be relatively high, such as 1 to 100 mg/mL or 10 to 100 mg/mL, or may be relatively low, such as 0.0001 to 1 mg/mL or 0.0001 to 0.1 mg/mL.

The protein formulation preferably has a pH of 4 to 9, more preferably 5 to 8. The above pH conditions are less likely to cause precipitation and exert less influence on protein activity. The pH of the protein formulation can be controlled using an acid, such as hydrochloric acid or sulfuric acid, or a base, such as sodium hydroxide or potassium hydroxide.

In addition to the protein and the composition for inhibiting protein denaturation, the protein formulation may contain optional components such as a pH adjuster, a thickener, an emulsifier, an inorganic salt, a preservative, a solvent, an excipient, a metal, or a filter aid. Specific examples of these components are as described above. The amount of these optional components is not limited and may be appropriately selected by a person skilled in the art. For example, it may be 0.001 to 90% by weight, preferably 0.01 to 50% by weight, more preferably 0.1 to 30% by weight, based on the protein formulation.

The protein formulation can be prepared by mixing the components in any order. After mixing the components, the mixture may be subjected to filtration or microorganism elimination by bringing it into contact with a porous material or by passing it through a filter. Moreover, the protein formulation in liquid form may be lyophilized to obtain a protein formulation powder.

<<Method for inhibiting protein denaturation>>

A method for inhibiting protein denaturation according to the present invention is characterized by including allowing a protein to coexist with the composition for inhibiting protein denaturation. Protein denaturation may be inhibited by the action of a NADES formed by the saccharide and the amine compound in the composition for inhibiting protein denaturation, although the present invention is not limited to this mechanism.

The method for allowing a protein to coexist with the composition for inhibiting protein denaturation is not limited as long as the protein, the saccharide, and the amine compound are allowed to coexist. Preferably, the composition for inhibiting protein denaturation in liquid form is mixed with a solution containing a protein to obtain a protein formulation, so that the protein coexists with the composition for inhibiting protein denaturation in the formulation.

Although the duration of coexistence of the protein with the composition for inhibiting protein denaturation may be determined according to the intended use or the storage period of the protein, it is preferably 30 minutes or longer, more preferably one hour or longer. The upper limit of the duration of coexistence is not limited, and the inhibition of protein denaturation can last for a long time. Generally, the upper limit is two years or shorter. The coexistence of the protein with the composition for inhibiting protein denaturation enables inhibition of protein denaturation even during long-term storage.

Although the temperature during the coexistence of the protein with the composition for inhibiting protein denaturation is not limited, it is preferably 0°C to 40°C, more preferably 4°C to 30°C. Protein denaturation can be inhibited even at a relatively high temperature at which protein denaturation can generally occur. The coexistence of the protein with the composition for inhibiting protein denaturation may be followed by an optional process such as purification of the protein or dilution.

The protein formulation according to the present invention can be applied to a wide range of uses depending on the type of protein contained therein. Examples of applicable uses include medical or pharmaceutical products such as antibody drugs, vaccines, diagnostic agents, hormone preparations, enzyme preparations, and in vitro diagnostic agents; foods and beverages such as nutritional supplements and seasonings; and industrial enzymes, detergents, cosmetics, quasi-drugs, animal feed, plant growth regulators, and biopreparations for plants.

EXAMPLES

The present invention is described below with reference to examples, but the present invention is not limited thereto. Hereinafter, the units "part(s)" and "%" refer to "part(s) by weight" and "% by weight", respectively, unless otherwise specified.

(1) Anti-human IFN$\gamma$ monoclonal antibody denaturation inhibition test

A saccharide and an amine compound were mixed at the concentrations indicated in Tables 1 to 13 to prepare a composition for inhibiting protein denaturation. The composition for inhibiting protein denaturation was mixed with a phosphate buffer containing a mouse monoclonal antibody (MAb-IFN$\gamma$-15, in-house product, LOT No. 010) against IFN$\gamma$ at a volume ratio of 9:1 in a 0.5 mL Eppendorf tube (Eppendorf, product No. 3810X) to prepare a protein formulation. The antibody concentration in the protein formulation was 70 $\mu$g/mL. Next, the protein formulation was heated at 73°C for five hours.

The heated protein formulation in an amount of 15 $\mu$L was dispensed into the wells of a 96-well microplate (Corning, Costar 3513), and then 135 $\mu$L of human IFN$\gamma$ (400 pg/mL) as an antigen was dispensed into the wells. After mixing with a 12-ch multi-channel pipette (Thermo Electron, Finnpippette), 100 $\mu$L of the mixture was taken and added to the wells of an immunomodule plate (Thermo Scientific, U16 maxisorp Loose Nunc-Immuno Module 469264) coated with MAb-IFN$\gamma$-15, thereby inducing an antigen-antibody reaction at room temperature for 1.5 hours. Thereafter, washing, addition of a labeled antibody, and measurement of the absorbance at a wavelength of 450 nm were performed according to standard procedures.

Separately, a reference test was performed as follows: 15 $\mu$L of a phosphate buffer was added to a 96-well microplate, to which 135 μL of human IFNγ (400 pg/mL) was then added. After mixing, 100 μL of the mixture was added to the wells of an immunomodule plate coated with MAb-IFNγ-15, and the absorbance was then measured.

The competitive inhibition rate was determined using the following equation. Tables 1 to 13 show the results.

Competitive inhibition rate (%) = (1- (absorbance in each example or comparative example/absorbance in reference test)) × 100

[Table 1]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-1-1 Control (unheated) | - | - | - | - | - | - | 87% |
| Comparative Example 1-1-2 Control (D-PBS) | - | - | - | - | - | - | -5% |
| Comparative Example 1-1-3 | Trehalose | 1.17 M | - | - | - | - | 72% |
| Comparative Example 1-1-4 | Sucrose | 1.17 M | - | - | - | - | 73% |
| Comparative Example 1-1-5 | Maltitol | 1.17 M | - | - | - | - | 75% |
| Comparative Example 1-1-6 | Isomaltitol | 1.17 M | - | - | - | | 71% |
| Comparative Example 1-1-7 | - | - | Choline chloride | 1.17 M | - | - | -11% |
| Comparative Example 1-1-8 | - | - | Arginine hydrochloride | 1.17 M | - | - | -14% |
| Comparative Example 1-1-9 | - | - | Urea | 1.17 M | - | - | -3% |
| Comparative Example 1-1-10 | - | - | Serine | 1.17 M | - | - | -5% |
| Comparative Example 1-1-11 | Sucrose | 1.17 M | Urea | 1.17 M | 1:1 | 2.34 M | 7% |
| Comparative Example 1-1-12 | Maltitol | 1.17 M | Urea | 1.17 M | 1:1 | 2.34 M | -1% |
| Example 1-1-1 | Trehalose | 1.17 M | Arginine hydrochloride | 1.17 M | 1:1 | 2.34 M | 89% |
| Example 1-1-2 | Trehalose | 1.17M | Serine | 1.17 M | 1:1 | 2.34 M | 80% |
| Example 1-1-3 | Sucrose | 1.17 M | Choline chloride | 1.17 M | 1:1 | 2.34 M | 75% |
| Example 1-1-4 | Sucrose | 1.17 M | Arginine hydrochloride | 1.17 M | 1:1 | 2.34 M | 82% |
| Example 1-1-5 | Maltitol | 1.17 M | Arginine hydrochloride | 1.17 M | 1:1 | 2.34 M | 88% |
| Example 1-1-6 | Isomaltitol | 1.17 M | Arginine hydrochloride | 1.17 M | 1:1 | 2.34 M | 90% |

In Comparative Example 1-1-1, the competitive inhibition rate of the unheated protein formulation containing no saccharide and no amine compound was measured and found to be 87%. In Comparative Example 1-1-2, the protein formulation containing no saccharide and no amine compound was heated. The competitive inhibition rate of the resulting protein formulation was found to be -5%. The competitive inhibition rates in Example 1-1-1 to Example 1-1-6 were higher than those in the corresponding comparative examples each using the same concentration of the same saccharide or amine compound alone. In Comparative Example 1-1-11 and Comparative Example 1-1-12 each using urea as an amine compound, the competitive inhibition rates were low even though the urea was combined with a saccharide.

[Table 2]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-2-1 Control (unheated) | - | - | - | - | - | - | 87% |
| Comparative Example 1-2-2 Control (D-PBS) | - | - | - | - | - | - | -4% |
| Comparative Example 1-2-3 | Trehalose | 0.91 M | - | - | - | - | 19% |
| Comparative Example 1-2-4 | Trehalose | 1.04 M | - | - | - | - | 47% |
| Comparative Example 1-2-5 | Trehalose | 1.17 M | - | - | - | - | 72% |
| Comparative Example 1-2-6 | | - | Arginine hydrochloride | 0.91 M | - | - | -13% |
| Comparative Example 1-2-7 | | | Arginine hydrochloride | 1.04 M | - | - | -10% |
| Comparative Example 1-2-8 | | - | Arginine hydrochloride | 1.17 M | - | - | -12% |
| Example 1-2-1 | Trehalose | 0.91 M | Arginine hydrochloride | 0.91 M | 1:1 | 1.82 M | 32% |
| Example 1-2-2 | Trehalose | 1.04 M | Arginine hydrochloride | 1.04 M | 1:1 | 2.08 M | 74% |
| Example 1-2-3 | Trehalose | 1.17 M | Arginine hydrochloride | 1.17 M | 1:1 | 2.34 M | 88% |

The competitive inhibition rates in Example 1-2-1 to Example 1-2-3 were higher than those in the corresponding comparative examples each using the same concentration of the same saccharide or amine compound alone.

[Table 3]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-3-1 Control (unheated) | - | - | - | - | - | - | 91% |
| Comparative Example 1-3-2 Control (D-PBS) | - | - | - | - | - | - | -3% |
| Example 1-3-1 | Maltitol | 1.46 M | Arginine hydrochloride | 0.37 M | 4:1 | 1.83 M | 95% |
| Example 1-3-2 | Maltitol | 1.22 M | Arginine hydrochloride | 0.61 M | 2:1 | 1.83 M | 88% |
| Example 1-3-3 | Maltitol | 0.91 M | Arginine hydrochloride | 0.91 M | 1:1 | 1.82 M | 50% |
| Example 1-3-4 | Maltitol | 1.87 M | Arginine hydrochloride | 0.47 M | 4:1 | 2.34 M | 92% |
| Example 1-3-5 | Maltitol | 1.57 M | Arginine hydrochloride | 0.79 M | 2:1 | 2.36 M | 94% |
| Example 1-3-6 | Maltitol | 1.17 M | Arginine hydrochloride | 1.17 M | 1:1 | 2.34 M | 89% |
| Example 1-3-7 | Maltitol | 0.77 M | Arginine hydrochloride | 1.54 M | 1:2 | 2.31 M | 52% |
| Example 1-3-8 | Maltitol | 1.05 M | Arginine hydrochloride | 1.05 M | 1:1 | 2.10 M | 81% |
| Example 1-3-9 | Maltitol | 1.19 M | Arginine hydrochloride | 1.19 M | 1:1 | 2.38 M | 90% |
| Example 1-3-10 | Maltitol | 1.35 M | Arginine hydrochloride | 1.35 M | 1:1 | 2.70 M | 92% |
| Example 1-3-11 | Maltitol | 1.53 M | Arginine hydrochloride | 1.53 M | 1:1 | 3.06 M | 88% |

EP 4 736 865 A1

In Example 1-3-1 to Example 1-3-11, the competitive inhibition rate was examined while varying the molar ratio of maltitol and arginine hydrochloride in light of the results of Example 1-1-1 to Example 1-1-6 in Table 1. As a result, high competitive inhibition rates were also observed at molar ratios of maltitol and arginine hydrochloride other than 1:1, as well as at the 1:1 molar ratio, as shown in Table 3.

[Table 4]

| | Saccharide | | Amine compound | | Saccharide Amine compound molar ratio | Total concentration of saccharide and amine compound | Competitive inhibition rate |
|---|---|---|---|---|---|---|---|
| | Type | Concentration | Type | Concentration | | | |
| Comparative Example 1-4-1 Control (unheated) | - | - | - | - | - | - | 92% |
| Comparative Example 1-4-2 Control (D-PBS) | - | - | - | - | - | - | -2% |
| Comparative Example 1-4-3 | Sucrose | 1.17 M | - | - | - | - | 69% |
| Comparative Example 1-4-4 | Trehalose | 1.17 M | - | - | - | - | 76% |
| Comparative Example 1-4-5 | Maltitol | 1.17 M | - | - | - | - | 70% |
| Comparative Example 1-4-6 | - | - | Potassium glutamate | 1.17 M | - | - | 7% |
| Comparative Example 1-4-7 | - | - | Proline | 1.17 M | - | - | -4% |
| Example 1-4-1 | Sucrose | 1.17 M | Potassium glutamate | 1.17 M | 1:1 | 2.34 M | 87% |
| Example 1-4-2 | Trehalose | 1.17 M | Potassium glutamate | 1.17 M | 1:1 | 2.34 M | 88% |
| Example 1-4-3 | Maltitol | 1.17 M | Potassium glutamate | 1.17 M | 1:1 | 2.34 M | 86% |
| Example 1-4-4 | Maltitol | 1.17 M | Proline | 1.17 M | 1:1 | 2.34 M | 90% |

The competitive inhibition rates in Example 1-4-1 to Example 1-4-4 were higher than those in the corresponding comparative examples each using the same concentration of the same saccharide or amine compound alone.

[Table 5]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-5-1 Control (unheated) | - | - | - | - | - | - | 93% |
| Comparative Example 1-5-2 Control (D-PBS) | - | - | - | - | - | - | 6% |
| Comparative Example 1-5-3 | Trehalose | 0.13 M | - | - | - | - | 11% |
| Comparative Example 1-5-4 | Trehalose | 0.39 M | - | - | - | - | 12% |
| Comparative Example 1-5-5 | Trehalose | 0.65 M | - | - | - | - | 21% |
| Comparative Example 1-5-6 | Trehalose | 0.91 M | - | - | - | - | 67% |
| Comparative Example 1-5-7 | - | - | Potassium glutamate | 0.13 M | - | - | 10% |
| Comparative Example 1-5-8 | - | - | Potassium glutamate | 0.39 M | - | - | 7% |
| Comparative Example 1-5-9 | - | - | Potassium glutamate | 0.65 M | - | - | 6% |
| Comparative Example 1-5-10 | - | - | Potassium glutamate | 0.91 M | - | - | 16% |
| Example 1-5-1 | Trehalose | 0.13 M | Potassium glutamate | 0.13 M | 1:1 | 0.26 M | 10% |
| Example 1-5-2 | Trehalose | 0.39 M | Potassium glutamate | 0.39 M | 1:1 | 0.78 M | 60% |
| Example 1-5-3 | Trehalose | 0.65 M | Potassium glutamate | 0.65 M | 1:1 | 1.30 M | 89% |
| Example 1-5-4 | Trehalose | 0.91 M | Potassium glutamate | 0.91 M | 1:1 | 1.82 M | 85% |

In Example 1-5-1 to Example 1-5-4, although the total concentration of trehalose and potassium glutamate was lower than that in Example 1-4-2, the competitive inhibition rates in these examples, like that in Example 1-4-2, were higher than those in the corresponding comparative examples each using the same concentration of the same saccharide or amine compound alone.

[Table 6]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-6-1 Control (unheated) | - | - | - | - | - | - | 90% |
| Comparative Example 1-6-2 Control (D-PBS) | - | - | - | - | - | - | 2% |
| Comparative Example 1-6-3 | Sucrose | 0.13 M | - | - | - | - | 0% |
| Comparative Example 1-6-4 | Sucrose | 0.39 M | - | - | - | - | 2% |
| Comparative Example 1-6-5 | Sucrose | 0.65 M | - | - | - | - | 4% |
| Comparative Example 1-6-6 | Sucrose | 0.91 M | - | - | - | - | 43% |
| Comparative Example 1-6-7 | | - | Potassium glutamate | 0.13 M | - | - | -2% |
| Comparative Example 1-6-8 | | - | Potassium glutamate | 0.65 M | - | - | -3% |
| Example 1-6-1 | Sucrose | 0.13 M | Potassium glutamate | 0.13 M | 1:1 | 0.26 M | 5% |
| Example 1-6-2 | Sucrose | 0.39 M | Potassium glutamate | 0.39 M | 1:1 | 0.78 M | 37% |
| Example 1-6-3 | Sucrose | 0.65 M | Potassium glutamate | 0.65 M | 1:1 | 1.30 M | 84% |
| Example 1-6-4 | Sucrose | 0.91 M | Potassium glutamate | 0.91 M | 1:1 | 1.82 M | 84% |

EP 4 736 865 A1

19

In Example 1-6-1 to Example 1-6-4, although the total concentration of sucrose and potassium glutamate was lower than that in Example 1-4-1, the competitive inhibition rates of these examples, like that in Example 1-4-1, were higher than those in the corresponding comparative examples each using the same concentration of the same saccharide or amine compound alone. Moreover, in the examples, the competitive inhibition rate tended to increase as the total concentration of the saccharide and the amine compound increased.

[Table 7]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-7-1 Control (unheated) | - | - | - | - | - | - | 91% |
| Comparative Example 1-7-2 Control (D-PBS) | - | - | - | - | - | - | -2% |
| Example 1-7-1 | Maltitol | 0.83 M | Potassium glutamate | 0.21 M | 4:1 | 1.04 M | 84% |
| Example 1-7-2 | Maltitol | 0.69 M | Potassium glutamate | 0.35 M | 2:1 | 1.04 M | 84% |
| Example 1-7-3 | Maltitol | 0.52 M | Potassium glutamate | 0.52 M | 1:1 | 1.04 M | 72% |
| Example 1-7-4 | Maltitol | 0.35 M | Potassium glutamate | 0.7 M | 1:2 | 1.05 M | 39% |
| Example 1-7-5 | Maltitol | 0.21 M | Potassium glutamate | 0.84 M | 1:4 | 1.05 M | 18% |
| Example 1-7-6 | Maltitol | 1.87 M | Potassium glutamate | 0.47 M | 4:1 | 2.34 M | 91% |
| Example 1-7-7 | Maltitol | 1.56 M | Potassium glutamate | 0.78 M | 2:1 | 2.34 M | 92% |
| Example 1-7-8 | Maltitol | 1.17M | Potassium glutamate | 1.17 M | 1:1 | 2.34 M | 86% |
| Example 1-7-9 | Maltitol | 0.78 M | Potassium glutamate | 1.56 M | 1:2 | 2.34 M | 85% |
| Example 1-7-10 | Maltitol | 0.47 M | Potassium glutamate | 1.87 M | 1:4 | 2.34 M | 86% |

In Example 1-7-1 to Example 1-7-10, the competitive inhibition rate was examined while varying the molar ratio of maltitol and potassium glutamate in light of the results of Example 1-4-1 to Example 1-4-3 in Table 4. As a result, a competitive inhibition rate higher than that in Comparative Example 1-7-2 was also observed at molar ratios of maltitol and potassium glutamate ranging from 4:1 to 1:4, as well as at the 1:1 molar ratio, as shown in Table 7. In Example 1-7-1 to Example 1-7-5 and Example 1-7-6 to Example 1-7-10, in which the total concentration of the saccharide and the amine compound was constant, the competitive inhibition rate tended to increase as the concentration of the saccharide increased.

[Table 8]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-8-1 Control (unheated) | - | - | - | - | - | - | 83% |
| Comparative Example 1-8-2 Control (D-PBS) | - | - | - | - | - | - | -10% |
| Example 1-8-1 | Maltitol | 0.45 M | Potassium glutamate | 0.45 M | 1:1 | 0.90 M | 27% |
| Example 1-8-2 | Maltitol | 0.45 M | Potassium glutamate | 0.90 M | 1:2 | 1.35 M | 41% |
| Example 1-8-3 | Maltitol | 0.45 M | Potassium glutamate | 1.80 M | 1:4 | 2.25 M | 80% |

In Example 1-8-1 to Example 1-8-3, as in Example 1-7-1 to Example 1-7-10, a competitive inhibition rate higher than that in Comparative Example 1-8-2 was also observed at molar ratios of maltitol and potassium glutamate ranging from 1:2 to 1:4, as well as at the 1:1 molar ratio. In the examples, the competitive inhibition rate tended to increase as the concentration of the amine compound increased.

[Table 9]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-9-1 Control (unheated) | - | - | - | - | - | - | 89% |
| Comparative Example 1-9-2 Control (D-PBS) | - | - | - | - | - | - | -1% |
| Comparative Example 1-9-3 | Maltitol | 1.00 M | - | - | - | - | 15% |
| Comparative Example 1-9-4 | - | - | Potassium glutamate | 0.06 M | - | - | 2% |
| Comparative Example 1-9-5 | - | - | Potassium glutamate | 0.13 M | - | - | -5% |
| Comparative Example 1-9-6 | - | - | Potassium glutamate | 0.25 M | - | - | -2% |
| Comparative Example 1-9-7 | - | - | Potassium glutamate | 0.50 M | - | - | 4% |
| Comparative Example 1-9-8 | - | - | Potassium glutamate | 1.00 M | - | - | 9% |
| Example 1-9-1 | Maltitol | 1.00 M | Potassium glutamate | 0.06 M | 16:1 | 1.06 M | 51% |
| Example 1-9-2 | Maltitol | 1.00 M | Potassium glutamate | 0.13 M | 8:1 | 1.13 M | 78% |
| Example 1-9-3 | Maltitol | 1.00 M | Potassium glutamate | 0.25 M | 4:1 | 1.25 M | 86% |
| Example 1-9-4 | Maltitol | 1.00 M | Potassium glutamate | 0.50 M | 2:1 | 1.50 M | 87% |
| Example 1-9-5 | Maltitol | 1.00 M | Potassium glutamate | 1.00 M | 1:1 | 2.00 M | 84% |

The competitive inhibition rates in Example 1-9-1 to Example 1-9-5 were higher than those in the corresponding comparative examples each using the same concentration of the same saccharide or amine compound alone. In the examples, the competitive inhibition rate tended to increase as the concentration of the amine compound increased.

[Table 10]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-10-1 Control (unheated) | - | - | - | - | - | - | 91% |
| Comparative Example 1-10-2 Control (D-PBS) | - | - | - | - | - | - | 1% |
| Comparative Example 1-10-3 | Maltitol | 0.80 M | - | - | - | - | 7% |
| Comparative Example 1-10-4 | - | - | Potassium glutamate | 0.06 M | - | - | 5% |
| Comparative Example 1-10-5 | - | - | Potassium glutamate | 0.13 M | - | - | -4% |
| Comparative Example 1-10-6 | - | - | Potassium glutamate | 0.25 M | - | - | 0% |
| Comparative Example 1-10-7 | - | - | Potassium glutamate | 0.50 M | - | - | 0% |
| Comparative Example 1-10-8 | - | - | Potassium glutamate | 1.00 M | - | - | 12% |
| Example 1-10-1 | Maltitol | 0.80 M | Potassium glutamate | 0.06 M | 12.8:1 | 0.86 M | 23% |
| Example 1-10-2 | Maltitol | 0.80 M | Potassium glutamate | 0.13 M | 6.4:1 | 0.93 M | 44% |
| Example 1-10-3 | Maltitol | 0.80 M | Potassium glutamate | 0.25 M | 3.2:1 | 1.05 M | 57% |
| Example 1-10-4 | Maltitol | 0.80 M | Potassium glutamate | 0.50 M | 1.6:1 | 1.30 M | 87% |
| Example 1-10-5 | Maltitol | 0.80 M | Potassium glutamate | 1.00 M | 0.8:1 | 1,80 M | 81% |

The competitive inhibition rates in Example 1-10-1 to Example 1-10-5 were higher than those in the corresponding comparative examples each using the same concentration of the same saccharide or amine compound alone. In the examples, the competitive inhibition rate tended to increase as the concentration of the amine compound increased.

[Table 11]

| | Saccharide | | Amine compound | | Saccharide Amine compound molar ratio | Total concentration of saccharide and amine compound | Competitive inhibition rate |
|---|---|---|---|---|---|---|---|
| | Type | Concentration | Type | Concentration | | | |
| Comparative Example 1-11-1 Control (unheated) | - | - | - | - | - | - | 91% |
| Comparative Example 1-11-2 Control (D-PBS) | - | - | - | - | - | - | 1% |
| Comparative Example 1-11-3 | Maltitol | 0.30 M | - | - | - | - | -2% |
| Comparative Example 1-11-4 | | - | Potassium glutamate | 0.50 M | - | - | 0% |
| Comparative Example 1-11-5 | | - | Potassium glutamate | 1.00 M | - | - | 12% |
| Example 1-11-1 | Maltitol | 0.30 M | Potassium glutamate | 0.50 M | 1:1.67 | 0.80 M | 14% |
| Example 1-11-2 | Maltitol | 0.30 M | Potassium glutamate | 1.00 M | 1:3.3 | 1.30 M | 20% |

The competitive inhibition rates in Example 1-11-1 and Example 1-11-2 were higher than those in the corresponding comparative examples each using the same concentration of the same saccharide or amine compound alone.

[Table 12]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 1-12-1 Control (unheated) | - | - | - | - | - | - | 83% |
| Comparative Example 1-12-2 Control (D-PBS) | - | - | - | - | - | - | -2% |
| Comparative Example 1-12-3 | Trehalose | 0.26 M | - | - | - | - | 2% |
| Comparative Example 1-12-4 | Trehalose | 0.52 M | - | - | - | - | -3% |
| Comparative Example 1-12-5 | Trehalose | 0.78 M | - | - | - | - | 19% |
| Comparative Example 1-12-6 | Trehalose | 1.04 M | - | - | - | - | 65% |
| Example 1-12-1 | Trehalose | 0.26 M | Potassium glutamate | 0.26 M | 1:1 | 0.52 M | 6% |
| Example 1-12-2 | Trehalose | 0.52 M | Potassium glutamate | 0.52 M | 1:1 | 1.04 M | 52% |
| Example 1-12-3 | Trehalose | 0.78 M | Potassium glutamate | 0.78 M | 1:1 | 1.56 M | 67% |
| Example 1-12-4 | Trehalose | 1.04 M | Potassium glutamate | 1.04 M | 1:1 | 2.08 M | 75% |
| Example 1-12-5 | Trehalose | 0.26 M | Sodium glutamate | 0.26 M | 1:1 | 0.52 M | 16% |
| Example 1-12-6 | Trehalose | 0.52 M | Sodium glutamate | 0.52 M | 1:1 | 1.04 M | 52% |
| Example 1-12-7 | Trehalose | 0.78 M | Sodium glutamate | 0.78 M | 1:1 | 1.56 M | 75% |
| Example 1-12-8 | Trehalose | 1.04 M | Sodium glutamate | 1.04 M | 1:1 | 2.08 M | 78% |

In Example 1-12-1 to Example 1-12-8, the competitive inhibition rates were higher than those in the corresponding comparative examples each using the same concentration of the same saccharide alone. Moreover, Example 1-12-5 to Example 1-12-8 were performed under the same conditions as in Example 1-12-1 to Example 1-12-4, respectively, except that potassium glutamate was replaced with sodium glutamate, and the same tendency as in Example 1-12-1 to Example 1-12-4 was observed. In the examples, the competitive inhibition rate tended to increase as the total concentration of the saccharide and the amine compound increased.

[Table 13]

| | Hydrogen bond donor | | Hydrogen bond receptor | | Hydrogen bond donor:Hydrogen bond receptor molar ratio | Total concentration of hydrogen bond donor and hydrogen bond receptor | Competitive inhibition rate |
|---|---|---|---|---|---|---|---|
| | Type | Concentration | Type | Concentration | | | |
| Comparative Example 1-13-1 Control (unheated) | - | - | - | - | - | - | 91% |
| Comparative Example 1-13-2 Control (D-PBS) | - | - | - | - | - | - | -2% |
| Comparative Example 1-13-3 | Urea | 1.60 M | Choline chloride | 0.80 M | 2:1 | 2.40 M | -11% |
| Comparative Example 1-13-4 | Urea | 1.80 M | Choline chloride | 0.90 M | 2:1 | 2.70 M | -20% |
| Example 1-13-1 | Trehalose | 0.89 M | Arginine hydrochloride | 0.89 M | 1:1 | 1.78 M | 26% |
| Example 1-13-2 | Trehalose | 1.02 M | Arginine hydrochloride | 1.02 M | 1:1 | 2.04 M | 75% |
| Example 1-13-3 | Trehalose | 1.14 M | Arginine hydrochloride | 1.14 M | 1:1 | 2.28 M | 91% |

Urea possesses hydrogen atoms bound to nitrogen atoms having high electronegativity and therefore can function as a hydrogen bond donor. A deep eutectic solvent containing urea and choline chloride, also known as reline, has been reported to have a stabilizing effect on proteins, such as bovine serum albumin (Phys. Chem. Chem. Phys., 2022, 24, 5627-5637). Thus, this reline and a composition for inhibiting protein denaturation containing trehalose and arginine hydrochloride were compared for the antibody-stabilizing effect. As a result, in Comparative Example 1-13-3 and Comparative Example 1-13-4 each using a composition containing a combination of urea and choline chloride, the competitive inhibition rates were low. In contrast, in Example 1-13-1 to Example 1-13-3 each using the composition for inhibiting protein denaturation according to the present invention, high competitive inhibition rates were observed.

(2) Anti-human TNF$\alpha$ monoclonal antibody denaturation inhibition test 1

A saccharide and an amine compound were mixed at the concentrations indicated in Table 14 to prepare a composition for inhibiting protein denaturation. The composition for inhibiting protein denaturation was mixed with a phosphate buffer containing a mouse monoclonal antibody (MAb-TNF$\alpha$-5, in-house product, LOT No. 006) against human TNF$\alpha$ at a volume ratio of 9:1 in a 0.5 mL Eppendorf tube to prepare a protein formulation. The antibody concentration in the protein formulation was 70 $\mu$g/mL. Next, the protein formulation was heated at 73°C for five hours.

The heated protein formulation in an amount of 20 $\mu$L was dispensed into the wells of a 96-well plate, and then 130 $\mu$L of human TNF$\alpha$ (25 pg/mL) as an antigen was dispensed into the wells. After mixing with a 12-ch multi-channel pipette, 100 $\mu$L of the mixture was taken and added to the wells of an immunomodule plate coated with MAb-TNF$\alpha$-5, thereby inducing an antigen-antibody reaction at room temperature for 1.5 hours. Thereafter, washing, addition of a labeled antibody, and measurement of the absorbance at a wavelength of 450 nm were performed according to standard procedures.

Separately, a reference test was performed as follows: 20 $\mu$L of a phosphate buffer was added to a 96-well microplate, to which 130 $\mu$L of human TNF$\alpha$ (25 pg/mL) alone as an antigen was then added. After mixing, 100 $\mu$L of the mixture was added to the wells of an immunomodule plate coated with MAb-TNF$\alpha$-5, and the absorbance was then measured.

The competitive inhibition rate was determined using the following equation. Table 14 shows the results.

Competitive inhibition rate (%) = (1- (absorbance in each example or comparative example/absorbance in reference test)) $\times$ 100

[Table 14]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 2-1 Control (unheated) | - | - | - | - | - | - | 50% |
| Comparative Example 2-2 Control (D-PBS) | - | - | - | - | - | - | -6% |
| Comparative Example 2-3 | Trehalose | 0.65 M | - | - | - | - | 15% |
| Comparative Example 2-4 | Trehalose | 0.91 M | - | - | - | - | 32% |
| Comparative Example 2-5 | Trehalose | 1.17 M | - | - | - | - | 41% |
| Comparative Example 2-6 | - | - | Sodium glutamate | 0.65 M | - | - | 13% |
| Comparative Example 2-7 | - | - | Sodium glutamate | 0.91 M | - | - | 10% |
| Comparative Example 2-8 | - | - | Sodium glutamate | 1.17 M | - | - | 14% |
| Example 2-1 | Trehalose | 0.65 M | Sodium glutamate | 0.65 M | 1:1 | 1.30 M | 39% |
| Example 2-2 | Trehalose | 0.91 M | Sodium glutamate | 0.91 M | 1:1 | 1.82 M | 42% |
| Example 2-3 | Trehalose | 1.17 M | Sodium glutamate | 1.17 M | 1:1 | 2.34 M | 53% |

The competitive inhibition rates in Example 2-1 to Example 2-3 were higher than those in the corresponding comparative examples each using the same concentration of the same saccharide or amine compound alone. In the examples, the competitive inhibition rate tended to increase as the total concentration of the saccharide and the amine compound increased.

(3) Anti-human IFNα monoclonal antibody denaturation inhibition test 2

A saccharide and an amine compound were mixed at the concentrations indicated in Table 15 to prepare a composition for inhibiting protein denaturation. The composition for inhibiting protein denaturation was mixed with a phosphate buffer containing a mouse monoclonal antibody (MAb-IFNα-43, in-house product, LOT No. 003) against human IFNα at a volume ratio of 9:1 in a 0.5 mL Eppendorf tube to prepare a protein formulation. The antibody concentration in the protein formulation was 70 μg/mL. Next, the protein formulation was heated at 73°C for five hours.
The heated protein formulation in an amount of 20 μL was dispensed into the wells of a 96-well microplate, and then 130 μL of human IFNα (2500 pg/mL) as an antigen was dispensed into the wells. After mixing with a 12-ch multi-channel pipette, 100 μL of the mixture was taken and added to the wells of an immunomodule plate coated with MAb-IFNα-43, thereby inducing an antigen-antibody reaction at room temperature for 1.5 hours. Thereafter, washing, addition of a labeled antibody, and measurement of the absorbance at a wavelength of 450 nm were performed according to standard procedures. Separately, a reference test was performed as follows: 20 μL of a phosphate buffer was added to a 96-well microplate, to which 130 μL of human IFNα (2500 pg/mL) alone as an antigen was then added. After mixing, 100 μL of the mixture was added to the wells of an immunomodule plate coated with the same antibody against IFNα, and the absorbance was then measured. The competitive inhibition rate was determined using the following equation. Table 15 shows the results.

Competitive inhibition rate (%) = (1- (absorbance in each example or comparative example/absorbance in reference test)) × 100

[Table 15]

| | Saccharide | | Amine compound | | Saccharide:Amine compound molar ratio | Total concentration of saccharide and amine compound | Competitive inhibition rate |
|---|---|---|---|---|---|---|---|
| | Type | Concentration | Type | Concentration | | | |
| Comparative Example 3-1 Control (unheated) | - | - | - | - | - | - | 91% |
| Comparative Example 3-2 Control (D-PBS) | - | - | - | - | - | - | -4% |
| Comparative Example 3-3 | Trehalose | 0.39 M | - | - | - | - | 12% |
| Comparative Example 3-4 | Trehalose | 0.65 M | - | - | - | - | 12% |
| Comparative Example 3-5 | Trehalose | 0.91 M | - | - | - | - | 58% |
| Comparative Example 3-6 | Trehalose | 1.17 M | - | - | - | - | 81% |
| Comparative Example 3-7 | - | - | Sodium glutamate | 0.39 M | - | - | -9% |
| Comparative Example 3-8 | - | - | Sodium glutamate | 0.65 M | - | - | 4% |
| Comparative Example 3-9 | - | - | Sodium glutamate | 0.91 M | - | - | 26% |
| Comparative Example 3-10 | - | - | Sodium glutamate | 1.17 M | - | - | 47% |
| Example 3-1 | Trehalose | 0.39 M | Sodium glutamate | 0.39 M | 1:1 | 0.78 M | 37% |
| Example 3-2 | Trehalose | 0.65 M | Sodium glutamate | 0.65 M | 1:1 | 1.30 M | 78% |
| Example 3-3 | Trehalose | 0.91 M | Sodium glutamate | 0.91 M | 1:1 | 1.82 M | 85% |
| Example 3-4 | Trehalose | 1.17 M | Sodium glutamate | 1.17 M | 1:1 | 2.34 M | 89% |

The competitive inhibition rates in Example 3-1 to Example 3-4 were higher than those in the corresponding comparative examples each using the same concentration of the same saccharide or amine compound alone. In the examples, the competitive inhibition rate tended to increase as the total concentration of the saccharide and the amine compound increased.

(4) LDH denaturation inhibition test

A saccharide and an amine compound were mixed at the concentrations indicated in Table 16 to prepare a composition for inhibiting protein denaturation. The composition for inhibiting protein denaturation was mixed with a potassium phosphate buffer containing a lactate dehydrogenase (LDH) (Oriental Yeast Co., Ltd., Product No. 46776003) at a volume ratio of 9:1 in a 0.5 mL Eppendorf tube to prepare a protein formulation. The lactate dehydrogenase concentration in the protein formulation was 50 $\mu$g/mL. Next, the protein formulation was heated at 70°C for two hours. The enzymatic activity of the heated protein formulation was measured with a kit (Roche, product No. 11644793001). Table 16 shows the results.

[Table 16]

| | Saccharide | | Amine compound | | Saccharide:Amine compound molar ratio | Total concentration of saccharide and amine compound | Remaining enzymatic activity (%) |
| --- | --- | --- | --- | --- | --- | --- | --- |
| | Type | Concentration | Type | Concentration | | | |
| Comparative Example 4-1-1 Control (unheated) | - | - | - | - | - | - | 100 |
| Comparative Example 4-1-2 Control (heated) | - | - | - | - | - | - | 0 |
| Comparative Example 4-1-3 | Trehalose | 1.17 M | - | - | - | - | 5.1 |
| Comparative Example 4-1-4 | Maltitol | 1.17 M | - | - | - | - | 5.1 |
| Comparative Example 4-1-5 | Sucrose | 1.17 M | - | - | - | - | 1.5 |
| Comparative Example 4-1-6 | - | - | Potassium glutamate | 1.17 M | - | - | 8.9 |
| Example 4-1-1 | Trehalose | 0.78 M | Potassium glutamate | 0.78 M | 1:1 | 1.56 M | 70.2 |
| Example 4-1-2 | Trehalose | 0.91 M | Potassium glutamate | 0.91 M | 1:1 | 1.82 M | 71.8 |
| Example 4-1-3 | Trehalose | 1.04 M | Potassium glutamate | 1.04 M | 1:1 | 2.08 M | 72.9 |
| Example 4-1-4 | Trehalose | 1.17 M | Potassium glutamate | 1.17 M | 1:1 | 2.34 M | 95.7 |
| Example 4-1-5 | Maltitol | 1.17 M | Potassium glutamate | 1.17 M | 1:1 | 2.34 M | 83.5 |
| Example 4-1-6 | Sucrose | 1.17 M | Potassium glutamate | 1.17 M | 1:1 | 2.34 M | 82.9 |

The remaining enzymatic activities in Example 4-1-1 to Example 4-1-6 were higher than those in the corresponding comparative examples each using the same concentration of the same saccharide or amine compound alone.

A saccharide and an amine compound were mixed at the concentrations indicated in Table 17 to prepare a composition for inhibiting protein denaturation. The composition for inhibiting protein denaturation was mixed with a potassium phosphate buffer containing a lactate dehydrogenase (LDH) (Oriental Yeast Co., Ltd., Product No. 46776003) at a volume ratio of 9:1 in a 0.5 mL Eppendorf tube to prepare a protein formulation. The lactate dehydrogenase concentration in the protein formulation was 20 mg/mL. Next, the protein formulation was heated at 70°C for two hours. The enzymatic activity of the heated protein formulation was measured with a kit (Roche, product No. 11644793001). Table 17 shows the results.

[Table 17]

| | Saccharide | | Amine compound | | Saccharide:Amine compound | Concentration of saccharide and amine compound | Remaining enzymatic activity |
|---|---|---|---|---|---|---|---|
| | Type | Concentration | Type | Concentration | | | |
| Comparative Example 4-2-1 Control (unheated) | - | - | - | - | - | - | 100 |
| Comparative Example 4-2-2 Control (heated) | - | - | - | - | - | - | 2.2 |
| Comparative Example 4-2-3 | Trehalose | 0.91 M | - | - | - | - | 2.9 |
| Comparative Example 4-2-4 | Trehalose | 1.17 M | - | - | - | - | 4.7 |
| Comparative Example 4-2-5 | - | - | Sodium glutamate | 0.91 M | - | - | 2.5 |
| Comparative Example 4-2-6 | - | - | Sodium glutamate | 1.17 M | - | - | 3.2 |
| Example 4-2-1 | Trehalose | 0.91 M | Sodium glutamate | 0.91 M | 1:1 | 1.82 M | 89.8 |
| Example 4-2-2 | Trehalose | 1.17 M | Sodium glutamate | 1.17 M | 1:1 | 2.34 M | 108 |

The remaining enzymatic activities in Example 4-2-1 and Example 4-2-2 were higher than those in the corresponding comparative examples each using the same concentration of the same saccharide or amine compound alone. It was confirmed that the composition for inhibiting protein denaturation according to the present invention exhibited an effect of inhibiting a decrease in activity due to denaturation or aggregation caused by heat treatment, even for a high concentration (20 mg/mL) of lactate dehydrogenase.

(5) Cedar pollen allergen denaturation inhibition test 1

A saccharide and an amine compound were mixed at the concentrations indicated in Table 18 to prepare a composition for inhibiting protein denaturation. The composition for inhibiting protein denaturation was mixed with a phosphate buffer containing a cedar pollen allergen protein (Cry j1) (in-house product, LOT No. 180301) at a volume ratio of 9:1 in a 0.5 mL Eppendorf tube to prepare a protein formulation. The cedar pollen allergen protein concentration in the protein formulation was 8 $\mu$g/mL. Next, the protein formulation was heated at 40°C for 23 hours. The amount of the non-aggregated protein (ng/mL) in the heated protein formulation was measured with Cry j1 ELISA (in-house product). Table 18 shows the results.

[Table 18]

| | Saccharide | | Amine compound | | Saccharide:Amine compound molar ratio | Total concentration of saccharide and amine compound | Amount of non-aggregated protein (ng/mL) |
|---|---|---|---|---|---|---|---|
| | Type | Concentration | Type | Concentration | | | |
| Comparative Example 5-1 Control (unheated) | - | - | - | - | - | - | 2100 |
| Comparative Example 5-2 Control (heated) | - | - | - | - | - | - | 540 |
| Comparative Example 5-3 | Isomaltitol | 1.17 M | - | - | - | - | 1350 |
| Comparative Example 5-4 | - | - | Arginine hydrochloride | 1.17 M | - | - | 0 |
| Example 5-1 | Isomaltitol | 1.17 M | Arginine hydrochloride | 1.17 M | 1:1 | 2.34 M | 2800 |

The amount of the non-aggregated protein in Example 5-1 was higher than those in the comparative examples each using the same concentration of the same saccharide or amine compound alone.

(6) Cedar pollen allergen denaturation inhibition test 2

A saccharide and an amine compound were mixed at the concentrations indicated in Table 19 to prepare a composition for inhibiting protein denaturation. The composition for inhibiting protein denaturation was mixed with a phosphate buffer containing a cedar pollen allergen protein (Cry j2) (in-house product, LOT No. 180301) at a volume ratio of 9:1 in a 0.5 mL Eppendorf tube to prepare a protein formulation. The cedar pollen allergen protein concentration in the protein formulation was 2 μg/mL. Next, the protein formulation was heated at 40°C for four hours. The amount of the non-aggregated protein (ng/mL) in the heated protein formulation was measured with Cry j2 ELISA (in-house product). Table 19 shows the results.

[Table 19]

| | Saccharide | | Amine compound | | Saccharide:Amine compound molar ratio | Total concentration of saccharide and amine compound | Amount of non-aggregated protein (ng/mL) |
|---|---|---|---|---|---|---|---|
| | Type | Concentration | Type | Concentration | | | |
| Comparative Example 6-1 Control (unheated) | - | - | - | - | - | - | 470 |
| Comparative Example 6-2 Control (heated) | - | - | - | - | - | - | 30 |
| Comparative Example 6-3 | Isomaltitol | 1.17 M | - | - | - | - | 390 |
| Comparative Example 6-4 | - | - | Arginine hydrochloride | 1.17 M | - | - | 30 |
| Example 6-1 | Isomaltitol | 1.17 M | Arginine hydrochloride | 1.17 M | 1:1 | 2.34 M | 480 |

The amount of the non-aggregated protein in Example 6-1 was higher than those in the comparative examples each using the same concentration of the same saccharide or amine compound alone.

(7) Cedar pollen allergen denaturation inhibition test 3

A saccharide and an amine compound were mixed at the concentrations indicated in Table 20 to prepare a composition for inhibiting protein denaturation. The composition for inhibiting protein denaturation was mixed with a phosphate buffer containing a cedar pollen allergen protein SBP (in-house product, Lot No.180301, 900 μg/mL) at a volume ratio of 9:1 in a 1.5 mL Eppendorf tube to prepare a protein formulation.

The protein formulation was added to a 96-well black plate in an amount of 98 μl/well, and then a PROTEOSTAT (registered trademark) protein aggregation assay reagent (Enzo), a reagent which binds to aggregated proteins to emit fluorescence, was added in an amount of 2 μl/well, followed by gentle mixing. Subsequently, the plate was shielded from light with aluminum foil and incubated in an incubator at 37°C. The plate was taken out to measure the fluorescence intensity at Ex 550 nm/Em 600 nm over time. The fluorescence intensity after one hour of incubation was taken as 100%, and an increase in the fluorescence intensity over time was measured. Table 20 shows the results as the mean ± standard deviation (%) of three measurements.

[Table 20]

| | Saccharide | | Amine compound | | Saccharide:Amine compound molar ratio | Total concentration of saccharide and amine compound | Amount of aggregated protein after each heat-treatment period at 37°C | | | | |
|---|---|---|---|---|---|---|---|---|---|---|---|
| | Type | Concentration | Type | Concentration | | | 1 hr | 24 hr | 48 hr | 72 hr | 144 hr |
| Comparative Example 7-1 | - | - | - | - | - | - | 100 ± 3 % | 519 ± 25.7 % | 818 ± 52 % | 1023 ± 54 % | 1758 ± 85 % |
| Comparative Example 7-2 | Trehalose | 0.39 M | - | - | - | - | 100 ± 3.3 % | 184 ± 6.4 % | 208 ± 8.0 % | 232 ± 9.0 % | 367 ± 7.0 % |
| Comparative Example 7-3 | Trehalose | 0.91 M | - | - | - | - | 100 ± 3.3 % | 148 ± 4.9 % | 163 ± 6.0 % | 176 ± 6.1 % | 239 ± 7.0 % |
| Comparative Example 7-4 | - | - | Sodium glutamate | 0.39 M | - | - | 100 ± 8.6 % | 213 ± 15.7 % | 246 ± 14 % | 287 ± 14 % | 396 ± 20 % |
| Comparative Example 7-5 | - | - | Sodium glutamate | 0.91 M | - | - | 100 ± 8.7 % | 170 ± 24 % | 202 ± 24 % | 217 ± 23 % | 254 ± 26 % |
| Example 7-1 | Trehalose | 0.39 M | Sodium glutamate | 0.39 M | 1:1 | 0.78 M | 100 ± 1.9 % | 98.2 ± 3.8 % | 111 ± 4.0 % | 118 ± 1.5 % | 123 ± 1.0 % |
| Example 7-2 | Trehalose | 0.91 M | Sodium glutamate | 0.91 M | 1:1 | 1.82 M | 100 ± 4.4 % | 78.3 ± 1.6 % | 81.3 ± 2.0 % | 84.3 ± 1.5 % | 111 ± 16 % |

In Comparative EExample 7-1, the incubation of the cedar pollen allergen protein in D-PBS at 37°C resulted in an increase in fluorescence intensity over time, confirming that protein aggregation gradually occurred. In Comparative Example 7-2 to Comparative Example 7-5, each using the saccharide or amine compound alone, a certain inhibitory effect on protein aggregation was observed. In Example 7-1 and Example 7-2, the fluorescence intensities were reduced to about 50% of those in Comparative Example 7-2 to Comparative Example 7-5, confirming a strong inhibitory effect on protein aggregation.

(8) Human IFNγ denaturation inhibition test

A saccharide and an amine compound were mixed at the concentrations indicated in Table 21 to prepare a composition for inhibiting protein denaturation. The composition for inhibiting protein denaturation was mixed with a phosphate buffer containing human IFN-γ (in-house product, LOT No. 109025) at a volume ratio of 99:1 in a 0.5 mL Eppendorf tube to prepare a protein formulation. The IFN-γ concentration in the protein formulation was 80 ng/mL. Next, the protein formulation was heated at 40°C for seven hours. The amount (pg/mL) of active IFN-γ in the heated protein formulation was measured with human IFN-γ ELISA (in-house product). Table 21 shows the results.

[Table 21]

| | Saccharide | | Amine compound | | Saccharide:Amine compound molar ratio | Total concentration of saccharide and amine compound | Amount of active protein (pg/mL) |
| | Type | Concentration | Type | Concentration | | | |
|---|---|---|---|---|---|---|---|
| Comparative Example 8-1 Control (unheated) | - | - | - | - | - | - | 2300 |
| Comparative Example 8-2 Control (heated) | - | - | - | - | - | - | 170 |
| Comparative Example 8-3 | Isomaltitol | 1.17 M | - | - | - | - | 610 |
| Comparative Example 8-4 | - | - | Arginine hydrochloride | 1.17 M | - | - | 0 |
| Example 8-1 | Isomaltitol | 1.17 M | Arginine hydrochloride | 1.17 M | 1:1 | 2.34 M | 4100 |

In Example 8-1, the amount of active IFN-$\gamma$ was larger than those in the comparative examples each using the same concentration of the same saccharide or amine compound alone.

(9) Human TNF$\alpha$ denaturation inhibition test 2

A saccharide and an amine compound were mixed at the concentrations indicated in Table 22 to prepare a composition for inhibiting protein denaturation. The composition for inhibiting protein denaturation was mixed with a phosphate buffer containing human TNF-$\alpha$ (in-house product, LOT No. 960506) at 99:1 by volume in a 0.5 mL Eppendorf tube to prepare a protein formulation. The human TNF-$\alpha$ concentration in the protein formulation was 10 $\mu$g/mL. Next, the protein formulation was subjected to 10 cycles of freeze-thawing including freezing at -80°C and subsequent thawing at room temperature. The amount (ng/mL) of active TNF-$\alpha$ in the protein formulation after heating was measured with human TNF-$\alpha$ ELISA (in-house product). Table 22 shows the results.

[Table 22]

|  | Saccharide | | Amine compound | | Saccharide:Amine compound molar ratio | Total concentration of saccharide and amine compound | Amount of active protein (ng/mL) | |
|---|---|---|---|---|---|---|---|---|
|  | Type | Concentration | Type | Concentration |  |  | Before freeze storage | After freeze-thawing |
| Comparative Example 9-1 Control (D-PBS) | - | - | - | - | - | - | 2700 | 14 |
| Comparative Example 9-2 | Isomaltitol | 1.17 M | - | - | - | - | 3900 | 1100 |
| Comparative Example 9-3 | - | - | Arginine hydrochloride | 1.17 M | - | - | 2300 | 0 |
| Example 9-1 | Isomaltitol | 1.17 M | Arginine hydrochloride | 1.17 M | 1:1 | 2.34 M | 4000 | 3400 |

In Example 9-1, the amount of active TNF-$\alpha$ was larger than those in the comparative examples each using the same concentration of the same saccharide or amine compound alone.

(10) Anti-human IFN$\gamma$ monoclonal antibody denaturation inhibition test

A saccharide and an amine compound were mixed at the concentrations indicated in Tables 23 to 26 to prepare a composition for inhibiting protein denaturation. The composition for inhibiting protein denaturation was mixed with a phosphate buffer containing a mouse monoclonal antibody (MAb-IFN$\gamma$-15, in-house product, LOT No. 010) against IFN$\gamma$ at a volume ratio of 9:1 in a 0.5 mL Eppendorf tube (Eppendorf, product No. 3810X) to prepare a protein formulation. The antibody concentration in the protein formulation was 70 $\mu$g/mL. In the tests in Tables 23 and 24, 0.01% to 0.5% (w/v) of polysorbate 80 or polysorbate 20 (both available from FUJIFILM Wako Pure Chemical Corporation) was added. Next, the protein formulation was heated at 73°C for five hours.

The heated protein formulation in an amount of 15 $\mu$L was dispensed into the wells of a 96-well microplate (Corning, Costar 3513), and then 135 $\mu$L of human IFN$\gamma$ (400 pg/mL) as an antigen was dispensed into the wells. After mixing with a 12-ch multi-channel pipette (Thermo Electron, Finnpippette), 100 $\mu$L of the mixture was taken and added to the wells of an immunomodule plate (Thermo Scientific, U16 maxisorp Loose Nunc-Immuno Module 469264) coated with MAb-IFN$\gamma$-15, thereby inducing an antigen-antibody reaction at room temperature for 1.5 hours. Thereafter, washing, addition of a labeled antibody, and measurement of the absorbance at a wavelength of 450 nm were performed according to standard procedures.

Separately, a reference test was performed as follows: 15 $\mu$L of a phosphate buffer was added to a 96-well microplate, to which 135 $\mu$L of human IFN$\gamma$ (400 pg/mL) was then added. After mixing, 100 $\mu$L of the mixture was added to the wells of an immunomodule plate coated with MAb-IFN$\gamma$-15, and the absorbance was then measured. The competitive inhibition rate was determined using the following equation. Tables 23 to 26 show the results.

Competitive inhibition rate (%) = (1- (absorbance in each example or comparative example/absorbance in reference test)) $\times$ 100

[Table 23]

| | Saccharide | | Amine compound | | Surfactant | | Saccharide: Amine compound molar ratio | Competitive inhibition rate |
|---|---|---|---|---|---|---|---|---|
| | Type | Concentration | Type | Concentration | Type | Concentration | | |
| Comparative Example 10-1 Control (unheated) | - | - | - | - | - | - | - | 93% |
| Comparative Example 10-2 Control (D-PBS) | - | - | - | - | - | - | - | 0% |
| Comparative Example 10-3 | Trehalose | 0.5 M | - | - | - | - | - | 1% |
| Comparative Example 10-4 | - | - | Sodium glutamate | 0.5 M | - | - | - | 3% |
| Comparative Example 10-5 | - | - | - | - | Polysorbate 80 | 0.05% (w/v) | - | 7% |
| Comparative Example 10-6 | - | - | - | - | Polysorbate 80 | 0.1% (w/v) | - | 12% |
| Comparative Example 10-7 | - | - | - | - | Polysorbate 80 | 0.2% (w/v) | - | 1% |
| Comparative Example 10-8 | - | - | - | - | Polysorbate 80 | 0.5% (w/v) | - | 3% |
| Example 10-1 | Trehalose | 0.5 M | Sodium glutamate | 0.5 M | - | - | 1:1 | 39% |
| Example 10-2 | Trehalose | 0.5 M | Sodium glutamate | 0.5 M | Polysorbate 80 | 0.05% (w/v) | 1:1 | 69% |
| Example 10-3 | Trehalose | 0.5 M | Sodium glutamate | 0.5 M | Polysorbate 80 | 0.1% (w/v) | 1:1 | 65% |
| Example 10-4 | Trehalose | 0.5 M | Sodium glutamate | 0.5 M | Polysorbate 80 | 0.2% (w/v) | 1:1 | 61% |
| Example 10-5 | Trehalose | 0.5 M | Sodium glutamate | 0.5 M | Polysorbate 80 | 0.5% (w/v) | 1:1 | 59% |

As shown in Table 23, the competitive inhibition rates of 0.5 M trehalose alone (Comparative Example 10-3) and 0.5 M sodium glutamate alone (Comparative Example 10-4) were 1% and 3%, respectively, whereas the competitive inhibition rate of a 0.5 M composition for inhibiting protein denaturation containing both components (Example 10-1) was as high as 39%, demonstrating a synergistic effect. Moreover, the competitive inhibition rate of polysorbate 80 alone was 1% to 12% (Comparative Example 10-5 to Comparative Example 10-8); in contrast, the competitive inhibition rate (i.e., antibody-stabilizing effect) was synergistically improved when combined with the 0.5 M composition for inhibiting protein denaturation (Example 10-2 to Example 10-5).

Here, the test for synergy in the protein denaturation inhibitory effect was performed using a "two-way repeated measures analysis of variance (ANOVA)" with the software "Stateel 4", which is provided as an appendix to 4Steps Excel Statistics, 4th Edition (Hisae Yanai, OMS Publishing).

[Table 24]

| | Saccharide | | Amine compound | | Surfactant | | Saccharide: Amine compound molar ratio | Competitive inhibition rate |
|---|---|---|---|---|---|---|---|---|
| | Type | Concentration | Type | Concentration | Type | Concentration | | |
| Comparative Example 11-1 Control (unheated) | - | - | - | - | - | - | - | 92% |
| Comparative Example 11-2 Control (D-PBS) | - | - | - | - | - | - | - | 1% |
| Comparative Example 11-3 | Trehalose | 0.5 M | - | - | - | - | - | -7% |
| Comparative Example 11-4 | - | - | Sodium glutamate | 0.5 M | - | - | - | 3% |
| Comparative Example 11-5 | - | - | - | - | Polysorbate 20 | 0.01% (w/v) | - | 6% |
| Comparative Example 11-6 | - | - | - | - | Polysorbate 20 | 0.02% (w/v) | - | 7% |
| Comparative Example 11-7 | - | - | - | - | Polysorbate 20 | 0.05% (w/v) | - | -1% |
| Comparative Example 11-8 | - | - | - | - | Polysorbate 20 | 0.1% (w/v) | - | 9% |
| Comparative Example 11-9 | - | - | - | - | Polysorbate 20 | 0.2% (w/v) | - | 10% |
| Example 11-1 | Trehalose | 0.5 M | Sodium glutamate | 0.5 M | - | - | 1:1 | 38% |
| Example 11-2 | Trehalose | 0.5 M | Sodium glutamate | 0.5 M | Polysorbate 20 | 0.01% (w/v) | 1:1 | 59% |
| Example 11-3 | Trehalose | 0.5 M | Sodium glutamate | 0.5 M | Polysorbate 20 | 0.02% (w/v) | 1:1 | 55% |
| Example 11-4 | Trehalose | 0.5 M | Sodium glutamate | 0.5 M | Polysorbate 20 | 0.05% (w/v) | 1:1 | 54% |
| Example 11-5 | Trehalose | 0.5 M | Sodium glutamate | 0.5 M | Polysorbate 20 | 0.1% (w/v) | 1:1 | 57% |

(continued)

| | Saccharide | | Amine compound | | Surfactant | | Saccharide: Amine compound molar ratio | Competitive inhibition rate |
|---|---|---|---|---|---|---|---|---|
| | Type | Concentration | Type | Concentration | Type | Concentration | | |
| Example 11-6 | Trehalose | 0.5 M | Sodium glutamate | 0.5 M | Polysorbate 20 | 0.2% (w/v) | 1:1 | 52% |

As shown in Table 24, the competitive inhibition rates (i.e., antibody-stabilizing effect) of the compositions for inhibiting protein denaturation containing polysorbate 20 (Example 11-2 to Example 11-6) were improved as compared to the composition for inhibiting protein denaturation containing no polysorbate 20 (Example 11-1).

[Table 25]

| | Saccharide | | Amine compound | | Saccharide:Amine compound molar ratio | Competitive inhibition rate |
|---|---|---|---|---|---|---|
| | Type | Concentration | Type | Concentration | | |
| Comparative Example 12-1 Control (unheated) | - | - | - | - | - | 93% |
| Comparative Example 12-2 Control (D-PBS) | - | - | - | - | - | 1% |
| Comparative Example 12-3 | Trehalose | 1 M | - | - | - | 64% |
| Comparative Example 12-4 | - | - | Sodium glutamate | 0.0625 M | - | 0% |
| Comparative Example 12-5 | - | - | Sodium glutamate | 0.125 M | - | -10% |
| Comparative Example 12-6 | - | - | Sodium glutamate | 0.25 M | - | -1% |
| Comparative Example 12-7 | - | - | Sodium glutamate | 0.5 M | - | -2% |
| Comparative Example 12-8 | - | - | Sodium glutamate | 1 M | - | 7% |
| Example 12-1 | Trehalose | 1 M | Sodium glutamate | 0.0625 M | 16:1 | 81% |
| Example 12-2 | Trehalose | 1 M | Sodium glutamate | 0.125 M | 8:1 | 83% |
| Example 12-3 | Trehalose | 1 M | Sodium glutamate | 0.25 M | 4:1 | 87% |
| Example 12-4 | Trehalose | 1 M | Sodium glutamate | 0.5 M | 2:1 | 88% |
| Example 12-5 | Trehalose | 1 M | Sodium glutamate | 1 M | 1:1 | 89% |

As shown in Table 25, compared with trehalose alone (Comparative Example 12-3) or sodium glutamate alone (Comparative Example 12-4 to Comparative Example 12-8), the compositions for inhibiting protein denaturation containing both components (Example 12-1 to Example 12-5) resulted in higher competitive inhibition rates, demonstrating a synergistic effect.

[Table 26]

| | Saccharide | | Amine compound | | Saccharide:Amine compound molar ratio | Competitive inhibition rate |
| --- | --- | --- | --- | --- | --- | --- |
| | Type | Concentration | Type | Concentration | | |
| Comparative Example 13-1 Control (unheated) | - | - | - | - | - | 91% |
| Comparative Example 13-2 Control (D-PBS) | - | - | - | - | - | 2% |
| Comparative Example 13-3 | Trehalose | 0.52 M | - | - | - | 10% |
| Comparative Example 13-4 | Trehalose | 0.78 M | - | - | - | 24% |
| Comparative Example 13-5 | Trehalose | 1.04 M | - | - | - | 81% |
| Comparative Example 13-6 | - | - | Sodium aspartate | 0.52 M | - | 3% |
| Comparative Example 13-7 | - | - | Sodium aspartate | 0.78 M | - | -5% |
| Comparative Example 13-8 | - | - | Sodium aspartate | 1.04 M | - | 10% |
| Example 13-1 | Trehalose | 0.52 M | Sodium aspartate | 0.52 M | 1:1 | 53% |
| Example 13-2 | Trehalose | 0.78 M | Sodium aspartate | 0.78 M | 1:1 | 85% |
| Example 13-3 | Trehalose | 1.04 M | Sodium aspartate | 1.04 M | 1:1 | 87% |
| Example 13-4 | Trehalose | 0.52 M | Sodium glutamate | 0.52 M | 1:1 | 38% |
| Example 13-5 | Trehalose | 0.78 M | Sodium glutamate | 0.78 M | 1:1 | 86% |
| Example 13-6 | Trehalose | 1.04 M | Sodium glutamate | 1.04 M | 1:1 | 87% |

As shown in Table 26, compared with trehalose alone (Comparative Example 13-3 to Comparative Example 13-5) or sodium aspartate alone (Comparative Example 13-6 to Comparative Example 13-8), the compositions for inhibiting protein denaturation containing both components (Example 13-1 to Example 13-3) resulted in higher competitive inhibition rates, demonstrating a synergistic effect. The use of sodium aspartate as an amine compound exhibited the same effect as sodium glutamate.

(11) Malate dehydrogenase denaturation inhibition test

A saccharide and an amine compound were mixed at the concentrations indicated in Table 27 to prepare a composition for inhibiting protein denaturation. The composition for inhibiting protein denaturation and water containing a microbe-derived recombinant malate dehydrogenase (rMDH, Oriental Yeast Co., Ltd.) at a concentration of 50 $\mu$g/mL were heated at 90°C for 2.5 hours. The activity of the malate dehydrogenase in the heated solution was measured with an MDH enzyme activity assay kit (BioAssay Systems). Table 27 shows the absorbances of the enzymatic reaction product measured over time.

[Table 27]

| | Saccharide | | Amine compound | | Saccharide:Amine compound molar ratio | Absorbance of enzymatic reaction product | | | |
|---|---|---|---|---|---|---|---|---|---|
| | Type | Concentration | Type | Concentration | | 5 min | 10 min | 30 min | 120 min |
| Comparative Example 13-1 Control (unheated) | - | - | - | - | - | 0.21 | 0.355 | 1.008 | 2.401 |
| Comparative Example 13-2 Control (Distilled water) | - | - | - | - | - | 0.09 | 0.117 | 0.261 | 0.831 |
| Comparative Example 13-3 | Trehalose | 0.91 M | - | - | - | 0.078 | 0.104 | 0.248 | 0.788 |
| Comparative Example 13-4 | Trehalose | 1.04 M | - | - | - | 0.085 | 0.119 | 0.283 | 0.876 |
| Comparative Example 13-5 | Trehalose | 1.17 M | - | - | - | 0.095 | 0.128 | 0.293 | 0.855 |
| Comparative Example 13-6 | - | - | Sodium glutamate | 0.91 M | - | 0.069 | 0.076 | 0.119 | 0.331 |
| Comparative Example 13-7 | - | - | Sodium glutamate | 1.04 M | - | 0.067 | 0.075 | 0.119 | 0.324 |
| Comparative Example 13-8 | - | - | Sodium glutamate | 1.17 M | - | 0.067 | 0.075 | 0.121 | 0.328 |
| Example 13-1 | Trehalose | 0.91 M | Sodium glutamate | 0.91 M | 1:1 | 0.228 | 0.286 | 0.529 | 1.2 |
| Example 13-2 | Trehalose | 1.04 M | Sodium glutamate | 1.04 M | 1:1 | 0.293 | 0.357 | 0.607 | 1.35 |
| Example 13-3 | Trehalose | 1.17 M | Sodium glutamate | 1.17 M | 1:1 | 0.4 | 0.486 | 0.832 | 1.899 |

The absorbances of the heated enzyme (Comparative Example 13-2) were clearly lower than those of the unheated enzyme (Comparative Example 13-1). The absorbances were observed to be much lower when sodium glutamate alone was added to the enzyme and then heated (Comparative Example 13-6 to Comparative Example 13-8). Moreover, almost no difference was observed between the absorbances of Comparative Example 13-2 and those of trehalose added alone (Comparative Example 13-3 to Comparative Example 13-5), indicating that the protective effect of trehalose alone was insufficient. In contrast, in Example 13-1 to Example 13-3, each using a composition for inhibiting protein denaturation containing trehalose and sodium glutamate, the change in absorbance over time was comparable to that in unheated Comparative Example 13-1, demonstrating a synergistic stabilizing effect.

(12) Anti-human IL-17A antibody denaturation inhibition test 1

A saccharide and an amine compound were mixed at the concentrations indicated in Table 28 to prepare a composition for inhibiting protein denaturation. The composition for inhibiting protein denaturation and an aqueous solution containing a humanized anti-human IL-17A antibody at a concentration of 1 mg/mL were heated at 73°C for five hours. The degree of white turbidity caused by denaturation and aggregation of the heated protein was evaluated based on the optical density at 660 nm. Table 28 shows the results.
Further, the reactivity of the heated antibody to the antigen (human IL-17A) was measured by competitive ELISA. Table 28 shows the measured competitive inhibition rates (%).

[Table 28]

| | Saccharide | | Amine compound | | Saccharide:Amine compound molar ratio | Turbidity (OD 660 nm) | Competitive inhibition rate (%) |
| --- | --- | --- | --- | --- | --- | --- | --- |
| | Type | Concentration | Type | Concentration | | | |
| Comparative Example 14-1 Control (unheated) | - | - | - | - | - | 0.037 | 88 |
| Comparative Example 14-2 Control (Distilled water) | - | - | - | - | - | 0.122 | 18 |
| Comparative Example 14-3 | Trehalose | 1 M | - | - | - | 0.243 | 42 |
| Comparative Example 14-4 | - | - | Sodium glutamate | 0.25 M | - | 0.193 | 16 |
| Comparative Example 14-5 | - | - | Sodium glutamate | 0.5 M | - | 0.338 | 5 |
| Comparative Example 14-6 | - | - | Sodium glutamate | 1 M | - | 0.411 | 24 |
| Example 14-1 | Trehalose | 1 M | Sodium glutamate | 0.25 M | 4:1 | 0.03 | 84 |
| Example 14-2 | Trehalose | 1 M | Sodium glutamate | 0.5 M | 2:1 | 0.04 | 82 |
| Example 14-3 | Trehalose | 1 M | Sodium glutamate | 1 M | 1:1 | 0.066 | 78 |

As shown in Table 28, the turbidity in Comparative Example 14-2 increased to about 3.3 times that in Comparative Example 14-1. This indicates that heating caused the antibody to aggregate and produce white turbidity. The amount of white turbidity and the absorbance further increased when trehalose alone (Comparative Example 14-3) or sodium glutamate alone (Comparative Example 14-4 to Comparative Example 14-6) was added. However, in the presence of a composition for inhibiting protein denaturation containing 1 M trehalose and various concentrations of sodium glutamate, the turbidity was reduced, depending on the concentration of sodium glutamate, to a level ranging from comparable to that in unheated Comparative Example 14-1 to about 1.8 times that level; therefore, aggregation of the antibody protein was inhibited (Example 14-1 to Example 14-3).

As shown in Table 28, the heating of the antibody reduced the competitive inhibition rate to 18% (Comparative Example 14-2). In the presence of 1 M trehalose, the antigen-binding activity decreased by about 50% (Comparative Example 14-3), while the antigen-binding activity further decreased when sodium glutamate alone was added (Comparative Example 14-4 to Comparative Example 14-6), indicating a correlation with an increase in the amount of white turbidity. However, in the presence of a composition for inhibiting protein denaturation containing trehalose and sodium glutamate (Example 14-1 to Example 14-3), the antigen-binding activity was comparable to that of the unheated antibody, demonstrating that the composition for inhibiting protein denaturation had a synergistic antibody-protecting effect compared to that obtained using the saccharide or amine compound alone. The antibody used in the test is an example of antibody formulations used in clinical practice, and the results show that the composition for inhibiting protein denaturation also exhibits a protective effect on antibody formulations used in clinical applications.

(13) Anti-human IL-17A antibody denaturation inhibition test 2

The same procedures as in Comparative Example 14-1 to Comparative Example 14-6 and Example 14-1 to Example 14-3 were performed, except that sodium aspartate was used as the amine compound instead of sodium glutamate. The degree of white turbidity caused by denaturation and aggregation of the heated protein was evaluated based on the optical density at 660 nm. Table 29 shows the results. Table 29 also shows the reactivity of the heated antibody to the antigen (human IL-17A) measured by competitive ELISA.

[Table 29]

| | Saccharide | | Amine compound | | Saccharide:Amine compound molar ratio | Turbidity (OD 660 nm) | Competitive inhibition rate (%) |
|---|---|---|---|---|---|---|---|
| | Type | Concentration | Type | Concentration | | | |
| Comparative Example 15-1 Control (unheated) | - | - | - | - | - | 0.04 | 84 |
| Comparative Example 15-2 Control (Distilled water) | - | - | - | - | - | 0.171 | 9 |
| Comparative Example 15-3 | Trehalose | 1 M | - | - | - | 0.185 | 47 |
| Comparative Example 15-4 | - | - | Sodium aspartate | 0.25 M | - | 0.211 | 22 |
| Comparative Example 15-5 | - | - | Sodium aspartate | 0.5 M | - | 0.252 | 10 |
| Comparative Example 15-6 | - | - | Sodium aspartate | 1 M | - | 0.277 | 23 |
| Example 15-1 | Trehalose | 1 M | Sodium aspartate | 0.25 M | 4:1 | 0.034 | 84 |
| Example 15-2 | Trehalose | 1 M | Sodium aspartate | 0.5 M | 2:1 | 0.041 | 83 |
| Example 15-3 | Trehalose | 1 M | Sodium aspartate | 1 M | 1:1 | 0.059 | 78 |

As shown in Table 29, the turbidity in Comparative Example 15-3 increased to about 4.3 times that in Comparative Example 15-1. This indicates that heating caused the antibody to aggregate and produce white turbidity. When trehalose alone (Comparative Example 15-3) or sodium aspartate alone (Comparative Example 15-4 to Comparative Example 15-6) was added, the amount of white turbidity increased compared to that in Comparative Example 15-2. However, in the presence of a composition for inhibiting protein denaturation containing trehalose and various concentrations of sodium aspartate, the turbidity was reduced, depending on the concentration of sodium aspartate, to a level ranging from comparable to that in unheated Comparative Example 15-1 to about 1.5 times that level; therefore, aggregation of the antibody protein was inhibited (Example 15-1 to Example 15-3).

As shown in Table 29, the heating of the antibody reduced the antigen-binding activity to 9% (Comparative Example 15-2). In the presence of 1 M trehalose, the antigen-binding activity decreased to 47% (Comparative Example 15-3), while the antigen-binding activity further decreased when sodium glutamate alone was added (Comparative Example 15-4 to Comparative Example 15-6), indicating a correlation with an increase in the amount of white turbidity. However, in the presence of a composition for inhibiting protein denaturation containing 1 M trehalose alone and sodium aspartate (Example 15-1 to Example 15-3), the antigen-binding activity was comparable to that of the unheated antibody, demonstrating that the composition for inhibiting protein denaturation had a synergistic antibody-protecting effect compared to that obtained using the saccharide or amine compound alone.

(14) Lactase denaturation inhibition test

A saccharide and an amine compound were mixed at the concentrations indicated in Table 30 to prepare a composition for inhibiting protein denaturation. The composition for inhibiting protein denaturation and an aqueous solution containing a lactase derived from the genus *Kluyveromyces* at a concentration of about 6 mg/mL were heated at 50°C or 60°C for two hours. The degree of white turbidity caused by denaturation and aggregation of the heated protein was evaluated based on the optical density at 660 nm. Table 30 shows the results.

Furthermore, the activity (remaining activity (%)) of the heated lactase relative to the activity of the unheated lactase was measured. Table 30 shows the results.

[Table 30]

| | Saccharide | | Amine compound | | Saccharide:Amine compound molar ratio | Turbidity after heating (OD 660 nm) | | Remaining activity after heating (%) | |
|---|---|---|---|---|---|---|---|---|---|
| | Type | Concentration | Type | Concentration | | 50°C | 60°C | 50°C | 60°C |
| Comparative Example 16-1 Control (unheated) | - | - | - | - | - | 0.02 | 0.02 | 100 | 100 |
| Comparative Example 16-2 Control (Buffer) | - | - | - | - | - | 1.9 | 1.3 | 2 | 2 |
| Example 16 | Trehalose | 1.17 M | Sodium glutamate | 1.17 M | 1:1 | 0.03 | 0.21 | 99 | 95 |
| (Buffer: 0.005% manganese chloride, 0.02 M potassium phosphate buffer) | | | | | | | | | |

As shown in Table 30, in Comparative Example 16-2, white turbidity was observed, and the remaining activity after heating significantly decreased compared to that in Comparative Example 16-1. In contrast, in Example 16, white turbidity was inhibited, and the remaining activity was improved.

(15) 6-α-Glucanotransferase denaturation inhibition test 1

A saccharide and an amine compound were mixed at the concentrations indicated in Table 31 to prepare a composition for inhibiting protein denaturation. The composition for inhibiting protein denaturation and an aqueous solution containing a 6-α-glucanotransferase derived from the genus *Geobacillus* at a concentration of about 25 mg/mL were heated at 70°C for two hours. The degree of white turbidity caused by denaturation and aggregation of the heated protein was evaluated based on the optical density at 660 nm. Table 31 shows the results.

Furthermore, the activity (remaining activity (%)) of the heated 6-α-glucanotransferase relative to the activity of the unheated 6-α-glucanotransferase was measured. Table 31 shows the results.

EP 4 736 865 A1

[Table 31]

| | Saccharide | | Amine compound | | Saccharide:Amine compound molar ratio | Turbidity after heating (OD 660 nm) | Remaining activity after heating (%) |
|---|---|---|---|---|---|---|---|
| | Type | Concentration | Type | Concentration | | 70°C | 70°C |
| Comparative Example 17-1 Control (unheated) | - | - | - | - | - | 1.4 | 100 |
| Comparative Example 17-2 Control (Buffer) | | | - | - | - | 8.3 | 2 |
| Example 17 | Trehalose | 1.17 M | Sodium glutamate | 1.17 M | 1:1 | 5.3 | 76 |
| (Buffer: 0.05% triton, 0.01 M potassium phosphate buffer) | | | | | | | |

As shown in Table 31, in Comparative Example 17-2, white turbidity was observed, and the remaining activity after heating significantly decreased compared to that in Comparative Example 17-1. In contrast, in Example 17, white turbidity was inhibited, and the remaining activity was improved.

(16) 6-α-Glucanotransferase denaturation inhibition test 2

A saccharide and an amine compound were mixed to prepare a composition for inhibiting protein denaturation. The composition for inhibiting protein denaturation and an aqueous solution containing a 6-α-glucanotransferase derived from the genus *Geobacillus* at a concentration of about 25 mg/mL were lyophilized to prepare a powder satisfying the amounts indicated in Table 32. The lyophilized powder was incubated in an incubator at 70°C for two days. The state of the powder was maintained even after storage, with no change in appearance due to moisture absorption. Thereafter, the activity (remaining activity (%)) of the incubated 6-α-glucanotransferase relative to the activity of the unheated 6-α-glucanotransferase was measured. Table 32 shows the results.

[Table 32]

| | Saccharide | | Amine compound | | Saccharide:Amine compound molar ratio | Remaining activity after heating (%) |
|---|---|---|---|---|---|---|
| | Type | Amount (in 1 kg of powder) | Type | Amount (in 1 kg of powder) | | |
| Comparative Example 18-1 Control (unheated) | - | - | - | - | - | 100 |
| Comparative Example 18-2 Control (without composition for inhibiting denaturation) | - | - | - | - | - | 53 |
| Example 18-1 | Trehalose | 1.64 mol | Sodium glutamate | 1.64 mol | 1:1 | 98 |
| Example 18-2 | Trehalose | 1.58 mol | Sodium glutamate | 1.58 mol | 1:1 | 83 |
| Example 18-3 | Trehalose | 1.39 mol | Sodium glutamate | 1.39 mol | 1:1 | 86 |

As shown in Table 32, the remaining activity after heating in Comparative Example 18-2 significantly decreased compared to that in Comparative Example 18-1. In contrast, in Example 18-1 to Example 18-3, the remaining activity was improved and restored to a level comparable to that in Comparative Example 18-1.

(17) 6-α-Glucanotransferase denaturation inhibition test 3

A saccharide and an amine compound were mixed to prepare a composition for inhibiting protein denaturation. The composition for inhibiting protein denaturation and an aqueous solution containing a 6-α-glucanotransferase derived from the genus *Geobacillus* at a concentration of about 45 mg/mL were lyophilized to prepare a powder satisfying the amounts indicated in Table 33. The lyophilized powder was stored in an incubator at a temperature of 40°C and a relative humidity of 75% for three months. The state of the powder was maintained even after storage, with no change in appearance due to moisture absorption. Thereafter, the activity (remaining activity (%)) of the incubated 6-α-glucanotransferase relative to the activity of the 6-α-glucanotransferase before incubation was measured. Table 33 shows the results.

[Table 33]

| | Saccharide | | Amine compound | | Saccharide:Amine compound molar ratio | Remaining activity after three-month storage (%) |
|---|---|---|---|---|---|---|
| | Type | Amount (in 1 kg of powder) | Type | Amount (in 1 kg of powder) | | |
| Comparative Example 19 Control (without composition for inhibiting denaturation) | - | - | - | - | - | 45 |
| Example 19-1 | Trehalose | 0.6 mol | Sodium glutamate | 0.6 mol | 1:1 | 98 |
| Example 19-2 | Trehalose | 0.31 mol | Sodium glutamate | 0.31 mol | 1:1 | 91 |
| Example 19-3 | Trehalose | 0.11 mol | Sodium glutamate | 0.11 mol | 1:1 | 84 |

As shown in Table 33, the remaining activity after there-month storage in Comparative Example 19 significantly decreased. In contrast, the remaining activity was improved in Example 19-1 to Example 19-3.

(18) Serine protease denaturation inhibition test

A saccharide and an amine compound were mixed at the concentrations indicated in Tables 34 and 35 to prepare a composition for inhibiting protein denaturation. The composition for inhibiting protein denaturation and an aqueous solution containing a serine protease derived from the genus *Bacillus* (Nagase Viita Co., Ltd. "Bioprase (registered trademark)") at a concentration of about 1 mg/mL were heated at 70°C for two hours. The activity (remaining activity (%)) of the heated enzyme relative to the activity of the heated enzyme was measured. Tables 34 and 35 show the results.
The enzymatic activity was measured using a fluorescently labelled casein included in an Amplite protease activity assay kit available from AAT Bioquest. Specifically, a heat-treated sample was diluted 60-fold with purified water, and the dilution was added to a 96-well black plate (3603) available from Coming in an amount of 50 μL/well. Next, the fluorescently labelled casein was diluted 100-fold with a 80 mM phosphate buffer (pH 7.5), and the dilution was added to the black plate in an amount of 50 μL/well. After 15-minute incubation at 37°C, the fluorescence intensity (Ex/Em: 490 nm/525 nm) was measured. Separately, an unheated protein-degrading enzyme powder was serially diluted two-fold from 50 μg/mL with purified water to prepare an aqueous solution, which was then added as standard.

[Table 34]

| | Saccharide | | Amine compound | | Saccharide:Amine compound molar ratio | Remaining enzymatic activity (%) |
|---|---|---|---|---|---|---|
| | Type | Concentration | Type | Concentration | | |
| Comparative Example 20-1 Control (unheated) | - | - | - | - | - | 100 |
| Comparative Example 20-2 Control (Distilled water) | - | - | - | - | - | 4 |
| Comparative Example 20-3 | Trehalose | 1 M | - | - | - | 14 |

(continued)

| | Saccharide | | Amine compound | | Saccharide:Amine compound molar ratio | Remaining enzymatic activity (%) |
|---|---|---|---|---|---|---|
| | Type | Concentration | Type | Concentration | | |
| Comparative Example 20-4 | - | - | Sodium glutamate | 0.25 M | 1:1 | 19 |
| Comparative Example 20-5 | - | - | Sodium glutamate | 0.5 M | - | 39 |
| Comparative Example 20-6 | - | - | Sodium glutamate | 1M | - | 55 |
| Example 20-1 | Trehalose | 1M | Sodium glutamate | 0.25 M | 4:1 | 59 |
| Example 20-2 | Trehalose | 1M | Sodium glutamate | 0.5 M | 2:1 | 72 |
| Example 20-3 | Trehalose | 1M | Sodium glutamate | 1M | 1:1 | 72 |

As shown in Table 34, the remaining activity in Comparative Example 20-2 significantly decreased. In contrast, the remaining activity was improved in Example 20-1 to Example 20-3. In Example 20-1 and Example 20-2, a statistically significant synergistic effect was observed.

[Table 35]

| | Saccharide | | Amine compound | | Saccharide:Amine compound molar ratio | Remaining enzymatic activity (%) |
|---|---|---|---|---|---|---|
| | Type | Concentration | Type | Concentration | | |
| Comparative Example 21-1 Control (unheated) | - | - | - | - | - | 100 |
| Comparative Example 21-2 Control (Distilled water) | - | - | - | - | - | 5 |
| Comparative Example 21-3 | Trehalose | 1 M | - | - | - | 3 |
| Comparative Example 21-4 | - | - | Sodium aspartate | 0.25 M | 1:1 | 13 |
| Comparative Example 21-5 | - | - | Sodium aspartate | 0.5 M | - | 6 |
| Comparative Example 21-6 | - | - | Sodium aspartate | 1M | - | 9 |
| Example 21-1 | Trehalose | 1M | Sodium aspartate | 0.25 M | 4:1 | 37 |

(continued)

| | Saccharide | | Amine compound | | Saccharide:Amine compound molar ratio | Remaining enzymatic activity (%) |
|---|---|---|---|---|---|---|
| | Type | Concentration | Type | Concentration | | |
| Example 21-2 | Trehalose | 1M | Sodium aspartate | 0.5 M | 2:1 | 49 |
| Example 21-3 | Trehalose | 1M | Sodium aspartate | 1M | 1:1 | 66 |

As shown in Table 35, the remaining activity in Comparative Example 21-2 significantly decreased. In contrast, the remaining activity was improved in Example 21-1 to Example 21-3. In Example 21-1, Example 21-2, and Example 21-3, a statistically significant synergistic effect was observed. The serine protease (Nagase Viita Co., Ltd., "Bioprase (registered trademark)") is utilized in the cosmetics field as a mask formulation to decompose and exfoliate aged keratin, and is also utilized in industrial applications, for example, in the production of detergents and various cleaning agents. The results in Tables 34 and 35 suggest that the composition for inhibiting protein denaturation is applicable to cosmetic mask formulations and cleaning agents.

**Claims**

1.  A composition for inhibiting protein denaturation, comprising:

    at least one saccharide selected from the group consisting of trehalose, sucrose, maltitol, and isomaltitol; and
    at least one amine compound selected from the group consisting of glutamic acid, aspartic acid, arginine, proline, serine, choline, and salts thereof.

2.  The composition for inhibiting protein denaturation according to claim 1, which inhibits protein denaturation caused by heating or freeze-thawing.

3.  The composition for inhibiting protein denaturation according to claim 1 or 2, which inhibits protein aggregation.

4.  The composition for inhibiting protein denaturation according to claim 1 or 2,
    wherein the protein is at least one selected from the group consisting of antibodies, cytokines, allergens, and enzymes.

5.  The composition for inhibiting protein denaturation according to claim 1 or 2,
    wherein the protein is at least one selected from the group consisting of anti-IFNα antibodies, anti-IFNγ antibodies, anti-TNFα antibodies, anti-IL-17A antibodies, and anti-IL-18 antibodies.

6.  The composition for inhibiting protein denaturation according to claim 1 or 2,
    wherein the protein is at least one selected from the group consisting of IFNα, IFNγ, IL-18, and TNFα.

7.  The composition for inhibiting protein denaturation according to claim 1 or 2,
    wherein the protein is a cedar pollen allergen.

8.  The composition for inhibiting protein denaturation according to claim 1 or 2,
    wherein the protein is at least one selected from the group consisting of lactate dehydrogenases, glycosyltransferases, saccharide-degrading enzymes, malate dehydrogenases, and protein-degrading enzymes.

9.  The composition for inhibiting protein denaturation according to claim 1 or 2,
    wherein the protein is a virus-derived protein or a bacteria-derived protein.

10. The composition for inhibiting protein denaturation according to claim 1 or 2,
    wherein the protein is a blood coagulation factor or albumin.

11. The composition for inhibiting protein denaturation according to claim 1 or 2, wherein a molar ratio of the saccharide to the amine compound is from 40:1 to 1:40.

12. The composition for inhibiting protein denaturation according to claim 1 or 2,
wherein the composition is in liquid form, and a total concentration of the saccharide and the amine compound is from 0.20 mol/L to 3.50 mol/L.

13. The composition for inhibiting protein denaturation according to claim 1 or 2, which inhibits protein denaturation synergistically compared to a protein denaturation inhibitory effect of the saccharide and a protein denaturation inhibitory effect of the amine compound.

14. A method for inhibiting protein denaturation, comprising
allowing a protein to coexist with the composition for inhibiting protein denaturation according to claim 1 or 2.

15. A protein formulation, comprising
a protein and the composition for inhibiting protein denaturation according to claim 1 or 2.

16. A method for inhibiting protein denaturation, comprising
allowing a protein to coexist with:

at least one saccharide selected from the group consisting of trehalose, sucrose, maltitol, and isomaltitol, and
at least one amine compound selected from the group consisting of glutamic acid, aspartic acid, arginine, proline, serine, choline, and salts thereof.

17. A protein formulation, comprising:

at least one saccharide selected from the group consisting of trehalose, sucrose, maltitol, and isomaltitol;
at least one amine compound selected from the group consisting of glutamic acid, aspartic acid, arginine, proline, serine, choline, and salts thereof; and
a protein.

## INTERNATIONAL SEARCH REPORT

| International application No. |
| --- |
| **PCT/JP2024/024412** |

| | |
| --- | --- |
| **A.** | **CLASSIFICATION OF SUBJECT MATTER** |

*A61K 38/00*(2006.01)i; *A61K 38/19*(2006.01)i; *A61K 38/43*(2006.01)i; *A61K 39/35*(2006.01)i; *A61K 39/395*(2006.01)i; *A61K 47/16*(2006.01)i; *A61K 47/18*(2017.01)i; *A61K 47/26*(2006.01)i; *A61P 43/00*(2006.01)i

FI: A61K38/00; A61K47/26; A61K47/18; A61K47/16; A61P43/00 105; A61K39/395 Y; A61K38/19; A61K38/43; A61K39/35

According to International Patent Classification (IPC) or to both national classification and IPC

| | |
| --- | --- |
| **B.** | **FIELDS SEARCHED** |

Minimum documentation searched (classification system followed by classification symbols)

A61K38/00; A61K38/19; A61K38/43; A61K39/35; A61K39/395; A61K47/16; A61K47/18; A61K47/26; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | JP 2020-506955 A (JOINT STOCK COMPANY "BIOCAD") 05 March 2020 (2020-03-05) claims, examples | 1-5, 11-17 |
| Y | | 1-17 |
| X | WO 2023/006055 A1 (SHANGHAI JUNSHI BIOSCIENCES CO., LTD.) 02 February 2023 (2023-02-02) claims, examples | 1-4, 11-17 |
| Y | | 1-17 |
| X | WO 2015/166885 A1 (EISAI R&D MANAGEMENT CO., LTD.) 05 November 2015 (2015-11-05) claims, examples | 1-3, 11-17 |
| Y | | 1-17 |

| | |
| --- | --- |
| ✓ Further documents are listed in the continuation of Box C. | ✓ See patent family annex. |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 September 2024** | **24 September 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
|---|
| **PCT/JP2024/024412** |

### C.   DOCUMENTS CONSIDERED TO BE RELEVANT

| Category* | Citation of document, with indication, where appropriate, of the relevant passages | Relevant to claim No. |
|---|---|---|
| X | FORNEY-STEVENS, KM. et al. Addition of Amino Acids to Further Stabilize Lyophilized Sucrose-Based Protein Formulations: I. Screening of 15 Amino Acids in Two Model Proteins. J. Pharm. Sci. 2016, vol. 105, issue 2, pp. 697-704<br>   particularly, abstract, p. 699, stability studies, p. 700, fig. 1. | 1-4, 8, 10-17 |
| Y | | 1-17 |
| X | JP 2005-516998 A (MERCK PATENT GMBH) 09 June 2005 (2005-06-09)<br>   claims, examples, tables 1-3 | 1, 4, 11-17 |
| Y | | 1-17 |
| X | JP 8-333277 A (HOECHST JAPAN LTD.) 17 December 1996 (1996-12-17)<br>   claims, examples, tables 3, 4 | 1, 2, 4, 10-17 |
| Y | | 1-17 |
| X | JP 11-130682 A (NIHON PHARMACEUTICAL CO., LTD.) 18 May 1999 (1999-05-18)<br>   claims, paragraph [0004], examples, tables 1, 2 | 1-4, 10-17 |
| Y | | 1-17 |
| X | JP 2016-535065 A (GEORGIA TECH RESEARCH CORPORATION) 10 November 2016 (2016-11-10)<br>   claims, examples, fig. 4, 10 | 1, 2, 9, 11-17 |
| Y | | 1-17 |
| X | CHEN, S. et al. Development of a simple assay system for protein-stabilizing efficiency based on hemoglobin protection against denaturation and measurement of the cooperative effect of mixing protein stabilizers. Biosci. Biotechnol. Biochem. 2016, vol. 80, no. 10, pp. 1874-1878<br>   particularly, abstract, p. 1874, right column, second paragraph, materials and methods, fig. 3., 4. | 1, 11-17 |
| Y | | 1-17 |
| Y | JP 2021-521231 A (MERCK PATENT GMBH) 26 August 2021 (2021-08-26)<br>   claims, examples, paragraphs [0153]-[0166], fig. 10 | 1-17 |
| Y | JP 2017-519777 A (AMGEN INC.) 20 July 2017 (2017-07-20)<br>   claims | 1-17 |
| Y | 中島滋ほか, グルタミン酸ナトリウムによる血漿中乳酸脱水素酵素の冷凍貯蔵中失活の抑制, 日本化学会誌, 1990, vol. 1990, no. 3, pp. 331-333, (NAKAJIMA, Shigeru et al. Prevention of Freeze Inactivation of Plasma Lactate Dehydrogenase with Sodium Glutamate. NIPPON KAGAKU KAISHI.)<br>   in particular, results, discussion | 1-17 |
| Y | 阿久澤良造ほか, 凍結および凍結乾燥によるStreptococcus lactis菌体内低温性プロティナーゼの変性と変性抑止剤, 日本獣医畜産大学研究報告, 1983, vol. 32, pp. 139-142, (AKUZAWA, Ryozo et al. Denaturation and Preservatives of Low-Temperature-Active Intracellular Proteinase from Streptococcus lactis by Freezing and Freeze-Drying. The bulletin of the Nippon Veterinary and Zootechnical College.)<br>   particularly, p. 140, right column, second paragraph to p. 141, left column, third paragraph, table 2. | 1-17 |

Form PCT/ISA/210 (second sheet) (July 2022)

## INTERNATIONAL SEARCH REPORT
### Information on patent family members

International application No.

**PCT/JP2024/024412**

| Patent document cited in search report | | | Publication date (day/month/year) | Patent family member(s) | | | Publication date (day/month/year) |
|---|---|---|---|---|---|---|---|
| JP | 2020-506955 | A | 05 March 2020 | WO | 2015/034924 | A1 | |
| | | | | claims, examples | | | |
| | | | | US | 2016/0220483 | A1 | |
| | | | | EP | 3041504 | A1 | |
| WO | 2023/006055 | A1 | 02 February 2023 | JP | 2024-528724 | A | |
| | | | | EP | 4376809 | A1 | |
| WO | 2015/166885 | A1 | 05 November 2015 | US | 2017/0189487 | A1 | |
| | | | | claims, examples | | | |
| | | | | EP | 3138575 | A1 | |
| JP | 2005-516998 | A | 09 June 2005 | US | 2005/0220758 | A1 | |
| | | | | claims, examples, tables 1-3 | | | |
| | | | | WO | 2003/066102 | A1 | |
| | | | | EP | 1471942 | A1 | |
| JP | 8-333277 | A | 17 December 1996 | US | 6084074 | A | |
| | | | | claims, examples, tables 3, 4 | | | |
| | | | | EP | 754463 | A2 | |
| JP | 11-130682 | A | 18 May 1999 | (Family: none) | | | |
| JP | 2016-535065 | A | 10 November 2016 | WO | 2015/034924 | A1 | |
| | | | | claims, examples, fig. 4, 10 | | | |
| | | | | US | 2016/0220483 | A1 | |
| | | | | EP | 3041504 | A1 | |
| JP | 2021-521231 | A | 26 August 2021 | WO | 2019/201894 | A1 | |
| | | | | claims, examples, pp. 41-44, fig. 10 | | | |
| | | | | US | 2021/0379186 | A1 | |
| | | | | EP | 3781192 | A1 | |
| JP | 2017-519777 | A | 20 July 2017 | WO | 2015/200027 | A1 | |
| | | | | claims | | | |
| | | | | US | 2017/0209582 | A1 | |
| | | | | EP | 3160511 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009525986 T

- JP 2022097600 A

**Non-patent literature cited in the description**

- *Phys. Chem. Chem. Phys.*, 2022, vol. 24, 5627-5637